# EUROPEAN PATENT SPECIFICATION

(11) **EP 0 284 288 B1**
(45) Date of publication and mention of the grant of the patent: **27.05.1992**
(21) Application number: 88302318.6
(22) Date of filing: 17.03.1988
(51) Int. Cl.: C07D 401/04, C07D 409/04, C07D 405/04, C07D 407/04, A61K 31/44, A61K 31/50, A61K 31/505

(54) **Disubstituted acetylenes bearing heteroaromatic and heterobicyclic groups having retinoid like activity**
Heteroaromatische und heterobicyclische Gruppen enthaltende disubstituierte Acetylene mit retinoidähnlicher Wirkung
Acétylènes disubstitués ayant des groupes hétéro-aromatiques et hétérocycliques à activité du type rétinoide

(30) Priority: 20.03.1987 US 28279
(43) Date of publication of application: 28.09.1988
(73) Proprietor: ALLERGAN, INC, Irvine, California 92715 (US)
(72) Inventor: Chandraratna, Roshantha A. S., El Toro, CA 92630 (US)
(74) Representative: Hutchins, Michael Richard

(56) References cited:
- EP-A- 0 176 034
- DE-A- 2 300 367
- CHEMICAL ABSTRACTS, vol. 95, no. 9, August 31, 1981, Columbus, Ohio, USA
- AMES, D.E.; BULL, D.; TAKUNDWA, C. "A convenient synthesis of ethynyl-N-heteroarenes ." page 782, column 2, abstract no. 80 892s
- CHEMICAL ABSTRACTS, vol. 98, no. 17, April 25, 1983, Columbus, Ohio, USA
- OTSUKA PHARMACEUTICAL CO., LTD. "Carbostyril derivatives." page 567, column 1, abstract no. 143 285f

## Description

### Background

This invention relates to novel compounds having retinoid-like activity. More specifically, the invention relates to compounds having an ethynylheteroaromatic acid portion and a second portion which is a tetrahydroquinazolinyl, thiochromanyl, or chromanyl group. The acid function may also be converted to an alcohol, aldehyde or ketone or derivatives thereof, or may be reduced to -CH₃. It is anticipated that the oxidation products of these compounds, particularly the oxides of the thiocromanyl compounds, will have activity similar to that of the parent compound.

### Related Art

Carboxylic acid derivatives useful for inhibiting the degeneration of cartilage of the general formula 4-(2-(4,4-dimethyl-6-X)-2-methylvinyl)benzoic acid where X is tetrahydroquinolinyl, chromanyl or thiochromanyl are disclosed in European Patent Application 0133795 published January 9, 1985. An article in J. Med. Chem., 27, 1516 (1984) by M. I. Dawson, et. al. discloses compounds similar to the ones claimed herein, the inventor using an acetylenic group in place of the methyl-substituted trans double bond shown in Dawson. Also, the aromatic moiety in Dawson is phenyl rather than the heteroaromatic group claimed herein. See also European Patent Application 176034A published April 2, 1986 where tetrahydronaphthalene compounds having an ethynylbenzoic acid group are disclosed.

### Summary of the Invention

This invention covers compounds of formula I 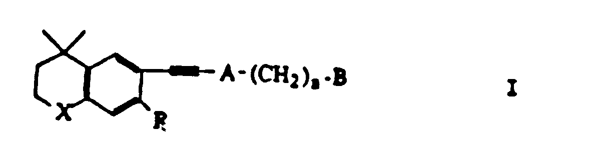
wherein X is S, O, or NR' where R' is hydrogen or lower alkyl; R is hydrogen or lower alkyl; A is pyridinyl, thienyl, furyl, pyridazinyl, pyrimidinyl or pyrazinyl; n is 0-5; and B is H, -COOH or a pharmaceutically acceptable salt, ester or amide thereof, -CH₂OH or an ether or ester derivative, or -CHO or an acetal derivative, or -COR₁ or a ketal derivative where R₁ is -(CH₂)ₘCH₃ where m is 0-4.

Particular and preferred compounds of the invention are as defined in the dependent claims appended hereto.

The compounds of this invention are useful for treating dermatoses, such as acne, Darier's disease, psoriasis, icthyosis, eczema, atopic dermatitis and epithelial cancers. These compounds are also useful in the treatment of arthritic diseases and other immunological disorders (e.g. lupus erythematosus), in promoting wound healing, in treating dry eye syndrome and in reversing the effects of sun damage to skin.

This invention also relates to a pharmaceutical formulation comprising a compound of formula I in admixture with a pharmaceutically acceptable excipient.

In another aspect, this invention relates to the process for making a compound of formula I which process comprises reacting a compound of formula II with a compound of formula III, in the presence of Pd(PQ₃)₄ (Q is phenyl) or a similar complex 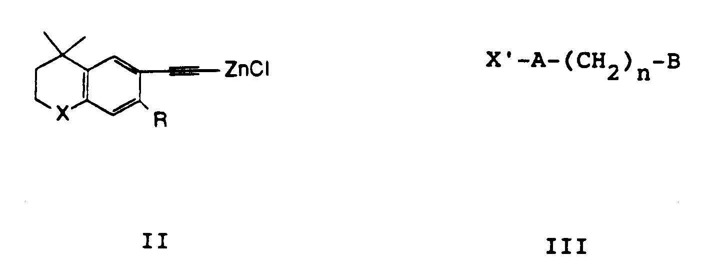
where Xʹ is a halogen, preferably I; n and A are the same as defined above; and B is H, or a protected acid, alcohol, aldehyde or ketone, giving the corresponding compound of formula I; or
deprotecting a protected acid, alcohol, aldehyde or ketone, of Formula I or
homologating a compound of the formula 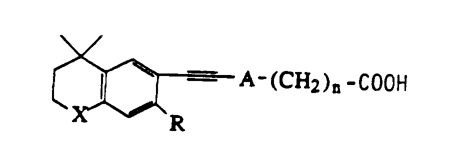
where n is 0-4 to give an acid of formula I; or
converting an acid of formula I to a salt; or
forming an acid addition salt;
converting an acid of formula I to an ester; or
converting an acid of formula I to an amide; or
reducing an acid of formula I to an alcohol or aldehyde; or
converting an alcohol of formula I to an ether or ester; or
oxidizing an alcohol of formula I to an aldehyde; or
converting an aldehyde of formula I to an acetal; or
converting a ketone of formula I to a ketal.

### General Embodiments

### Definitions

The term "ester" as used here refers to a compound falling within the definition of that term as classically used in organic chemistry. Where A is -COOH, this term covers the products derived from treatment of this function with alcohols. Where the ester is derived from compounds where A is -CH₂OH, this term covers compounds of the formula -CH₂OOCR where R is a particular substituted or unsubstituted aliphatic, aromatic or aliphatic-aromatic group.

The esters are derived from the saturated aliphatic alcohols or acids of ten or fewer carbon atoms or the cyclic or saturated aliphatic cyclic alcohols and acids of 5 to 10 carbon atoms, or the phenyl or C₁₋₆ alkylphenyl esters. Particularly preferred aliphatic esters are those derived from lower alkyl acids and alcohols. Here, and where ever else used, lower alkyl means having 1-6 carbon atoms.

The amides of the invention are the mono- and di-substituted amides derived from the saturated aliphatic radicals of ten or fewer carbon atoms or the cyclic or saturated aliphatic-cyclic radicals of 5 to 10 carbon atoms, or the mono- and di-substituted amides derived from the phenyl or lower alkylphenyl amines. Unsubstituted amides and C₁₋₆ alkylamides are preferred, the C₁₋₆ alkyl amides being particularly preferred.

Acetals and ketals includes the radicals of the formula -CK where K is (-OR)₂. Here, R is lower alkyl. Also, K may be -OR₁O- where R₁ is alkylene of 2-5 carbon atoms, straight chain or branched.

A pharmaceutically acceptable salt may be prepared for any compound of this invention having a functionality capable of forming such salt, for example an acid or an amine functionality. A pharmaceutically acceptable salt may be any salt which retains the activity of the parent compound and does not impart any deleterious or untoward effect on the subject to which it is administered and in the context in which it is administered.

Such a salt may be derived from any organic or inorganic acid or base. The salt may be a mono or polyvalent ion. Of particular interest where the acid function is concerned are the inorganic ions, sodium, potassium, calcium, and magnesium. Organic amine salts may be made with amines, particularly ammonium salts such as mono-, di- and trialkyl amines or ethanol amines. Salts may also be formed with caffeine, tromethamine and similar molecules. Where there is a nitrogen sufficiently basic as to be capable of forming acid addition salts, such may be formed with any inorganic or organic acids or alkylating agent such a methyl iodide. Preferred salts are those formed with inorganic acids such as hydrochloric acid, sulfuric acid or phosphoric acid. Any of a number of simple organic acids such as a mono-, di- or tri-acid may also be used.

The preferred compounds of this invention are those where the ethynyl group and the B group are attached to the 2 and 5 positions respectively of a pyridine ring (the 6 and 3 positions in the nicotinic acid nomenclature being equivalent to the 2/5 designation in the pyridine nomenclature) or the 5 and 2 positions respectively of a thiophene group respectively; n is 0, 1 or 2; and B is -COOH, an alkali metal salt or organic amine salt, or a lower alkyl ester, or -CH₂OH and the lower alkyl esters and ethers thereof. The more preferred compounds are:
ethyl 6-[2-(4,4-dimethylthiochroman-6-yl)ethynyl]nicotinoate;
6-[2-(4,4-dimethylthiochroman-6-yl)ethynyl]nicotinic acid;
6-[2-(4,4-dimethylchroman-6-yl)ethynyl]nicotinic acid; and
ethyl 6-[2-(4,4-dimethylchroman-6-yl)ethynyl]ate.

The compounds of this invention may be administered systemically or topically, depending on such considerations as the condition to be treated, need for site-specific treatment, quantity of drug to be administered, and similar considerations.

In the treatment of dermatoses, it will generally be preferred to administer the drug topically, though in certain cases such as treatment of severe cystic acne, oral administration may also be used. Any common topical formulation such as a solution, suspension, gel, ointment, or salve may be used. Preparation of such topical formulations are well described in the art of pharmaceutical formulations as exemplified, for example, Remington's Pharmaceutical Science, Edition 17, Mack Publishing Company, Easton, Pennsylvania. For topical application, these compounds could also be administered as a powder or spray, particularly in aerosol form.

If the drug is to be administered systemically, it may be confected as a powder, pill or tablet, or as a syrup or elixir for oral administration. For intravenous or intraperitoneal administration, the compound will be prepared as a solution or suspension capable of being administered by injection. In certain cases, it may be useful to formulate these compounds in suppository form or as an extended release formulation for deposit under the skin or intermuscular injection.

Other medicaments can be added to such topical formulation for such secondary purposes as treating skin dryness, providing protection against light; other medications for treating dermatoses, preventing infection, reducing irritation and inflammation.

Treatment of dermatoses or any other indications known or discovered to be susceptible to treatment by retinoic acid-like compounds will be effected by administration of the therapeutically effective dose of one or more compounds of the instant invention. A therapeutic concentration will be that concentration which effects reduction of the particular condition, or retards its expansion. In certain instances, the drug potentially could be used in a prophylactic manner to prevent onset of a particular condition. A given therapeutic concentration will vary from condition to condition and in certain instances may vary with the severity of the condition being treated and the patients susceptibility to treatment. Accordingly, a given therapeutic concentration will be best determined at the time and place through routine experimentation. However, it is anticipated that in the treatment of, for example, acne, or other such dermatoses, that a formulation containing between 0.001 and 5 percent by weight, preferably about 0.01 to 1%, will usually constitute a therapeutically effective concentration. If administered systemically, an amount between 0.01 and 100 mg per kg body weight per day, but preferably about 0.1 to 10 mg/kg, will effect a therapeutic result in most instances.

The retinoic acid-like activity of these compounds was confirmed through the classic measure of retinoic acid activity involving the effects of retinoic acid on ornithine decarboxylase. The original work on the correlation between retinoic acid and decrease in cell proliferation was done by Verma & Boutwell, Cancer Research, 1977, 37, 2196-2201. That reference discloses that ornithine decarboxylase (ODC) activity increased precedent to polyamine biosynthesis. It has been established elsewhere that increases in polyamine synthesis can be correlated or associated with cellular proliferation. Thus, if ODC activity could be inhibited, cell hyperproliferation could be modulated. Although all causes for ODC activity increase are unknown, it is known that 12-0-tetradecanoylphorbol-13-acetate (TPA) induces ODC activity. Retinoic acid inhibits this induction of ODC activity by TPA. The compounds of this invention also inhibit TPA induction of ODC as demonstrated by an assay essentially following the procedure set out in Cancer Res.: 1662-1670, 1975.

### Specific Embodiments

The compounds of this invention can be made by a number of different synthetic chemical pathways. To illustrate this invention, there is here outlined a series of steps which have been proven to provide the compounds of formula I when such synthesis is followed in fact and in spirit. The synthetic chemist will readily appreciate that the conditions set out here are specific embodiments which can be generalized to any and all of the compounds represented by formula I.

Compounds of formula I where X is -S- are prepared as per Reaction Scheme I. 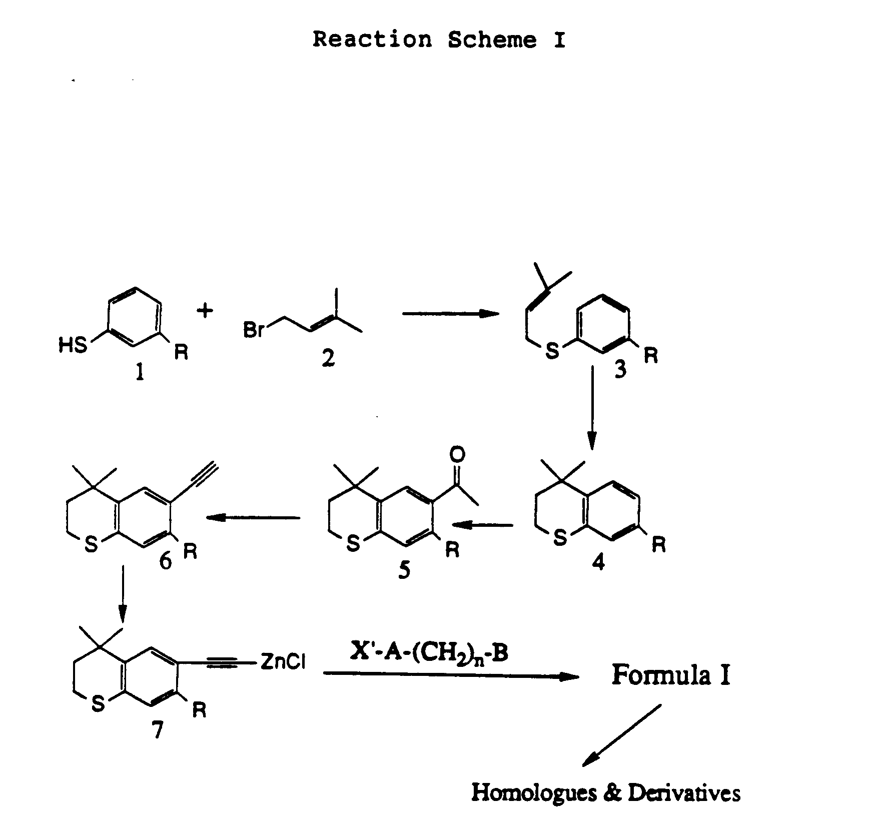
Here, R is hydrogen or a lower alkyl group, A is defined above, n is 0-5 and B is H, or a protected acid, alcohol, aldehyde or ketone. Xʹ is a halogen, preferably I.

Compounds of formula I were X is oxygen are prepared as per Reaction Scheme II. 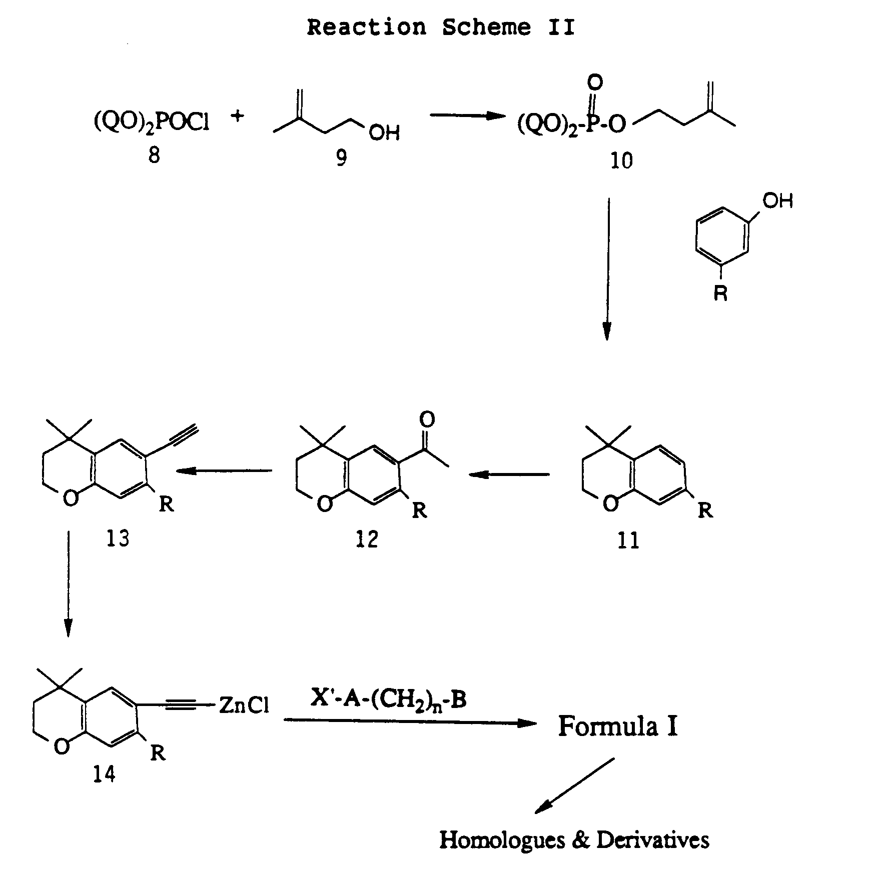
The definitions of R, n, A, B and Xʹ are the same here as in Scheme I.

In those instances where X is nitrogen, the sequence of steps outlined in Reaction Scheme III will serve to make such compounds. 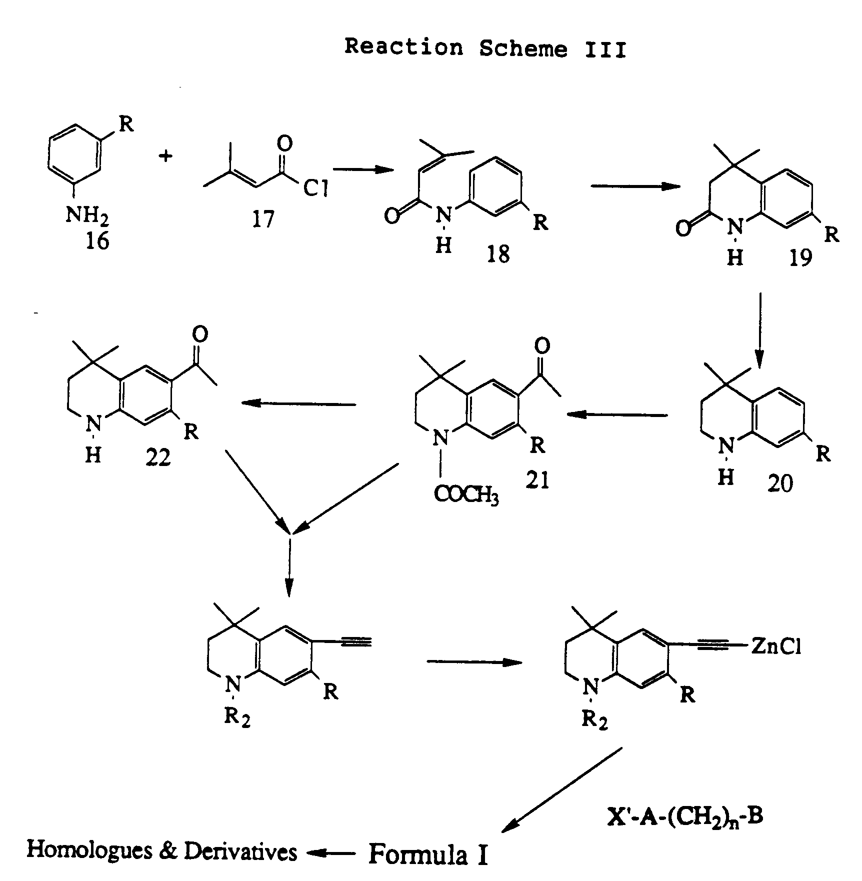
The definitions of R, n, A, B and Xʹ are the same here as in Scheme I and R₂ is hydrogen or -COCH₃.

A general description for making each of the compounds recited in the foregoing Reaction Schemes follows.

In Reaction Scheme I, the following generalized reaction conditions are applicable. The thiophenol of formula 1 is first treated with approximately an equimolar amount of a strong base such as an alkali metal hydroxide, preferably sodium hydroxide, in acetone at reflux. Refluxing is carried out for between 1 and 4 hours, preferably 2.5 hours, after which the solution is treated with an equimolar amount of formula 2, 1-bromo-3-methyl-2-butene (Aldrich), dissolved in acetone. Refluxing is continued for about 2 days after which the solution is stirred for another 24 hours at about room temperature effecting formation of formula 3. It is isolated by conventional means.

Ring closure is effected by treating the sulfide (compound 3), whose formation is described above, with phosphorous pentoxide in the presence of phosphoric acid under an inert atmosphere to give the thiochroman of formula 4. The sulfide is first dissolved in an inert solvent such as benzene or toluene, and then treated with a small excess of phosphorous pentoxide along with concentrated phosphoric acid. The solution is heated at reflux with stirring under an inert gas such as argon or nitrogen for up to 24 hours. The product is then recovered and purified by conventional means.

The ketone of formula 5 is obtained by treating the thiochroman with acetyl chloride in the presence of aluminum chloride. A suspension of the aluminum chloride in a polar inert solvent is prepared under an inert atmosphere and at reduced temperature, i.e., -10 to 10°C. The inert atmosphere may be argon or nitrogen, preferably argon. The reaction is conveniently carried out in a solvent such as methylene chloride. To the aluminum chloride suspension is added the thiochroman and acetyl chloride via a dropping funnel or similar device. About a 5% molar excess of acetyl chloride and 10% molar excess of aluminum chloride, relative to the thichroman material, is used. The reaction is effected with agitation (stirring) over 0.5-4 hours at a temperature between 10-50°C. Preferably the reaction is effected in about 2 hours at room temperature. Then the reaction is quenched with water and/or ice, the product extracted and further purified by distillation or some other appropriate means.

The acetylenic function of formula 6 is introduced by means of lithium diisopropylamide or a similar base at reduced temperature under an inert atmosphere. The reaction is carried out in an ether-type of solvent such as a dialkyl ether or a cyclic ether, for example, tetrahydrofuran or pyran.

More specifically, lithium diisopropylamide is generated in situ by mixing diisopropylamine in a dry solvent such as tetrahydrofuran, which is then cooled, to between -70 and -50°C under an inert atmosphere. An equimolar amount of an alkylithium compound such as n-butyl lithium in an appropriate solvent is then added at the reduced temperature and mixed for an appropriate time to permit formation of lithium diisopropylamide (LDA). The ketone of formula 5 (at least a 10% molar excess) is dissolved in the reaction solvent, the solution cooled to that of the LDA mixture, and added to that solution. After brief mixing, the solution is then treated with a dialkyl chlorophosphate, preferably diethyl chlorophosphate in about a 20% molar excess. The reaction solution is then gradually brought to room temperature. This solution is then added to a second lithium diisopropylamide solution which is prepared in situ using dry solvent all under an inert atmosphere, preferably argon, at reduced temperature (eg. -78°C). Thereafter, the reaction mixture is again warmed to room temperature where it is stirred for an extended period of time, preferably between 10 and 20 hours, most preferably about 15 hours. The solution is then acidified and the product recovered by conventional means.

Formula 7 compounds are prepared under conditions which exclude water and oxygen. A dry, ether-type solvent such as dialkyl ether or a cyclic ether such as a furan or pyran, particularly a tetrahydrofuran, may be used as the solvent. A solution of formula 6 is first prepared under an inert atmosphere such as argon or nitrogen, and then a strong base such as n-butyl lithium is added (in about a 10% molar excess). This reaction is begun at a reduced temperature of between -10° and +10°C, preferably about 0°C. The reaction mixture is stirred for a short period, between 30 minutes and 2 hours, and then treated with about a 10% molar excess of fused zinc chloride dissolved in the reaction solvent. This mixture is stirred for an additional 1-3 hours at about the starting temperature, then the temperature is increased to about ambient temperature for 10-40 minutes.

Where a protected heteroaromatic compound is needed to couple with formula 7 compounds, such may be prepared from their corresponding acids, alcohols, ketones or aldehydes. These starting materials, the protected acids, alcohols aldehydes or ketones, are all available from chemical manufacturers or can be prepared by published methods. Acids are esterified by refluxing the acid in a solution of the appropriate alcohol in the presence of thionyl chloride. Refluxing for 2-5 hours provides the desired ester. Alternatively, the acid can be condensed with the appropriate alcohol in the presence of dicyclohexylcarbodiimide and dimethylaminopyridine. The ester is recovered and purified by conventional means. Acetals and ketals are readily made by the method described in March, "Advanced Organic Chemistry," 2nd Edition, McGraw-Hill Book company, p 810). Alcohols, aldehydes and ketones all may be protected by forming respectively, ethers and esters, acetals or ketals by known methods such as those described in McOmie, Plenum Publishing Press, 1973 and Protecting Groups, Ed. Greene, John Wiley & Sons, 1981.

To increase the value of n before effecting a coupling reaction, where such compounds are not available from a commercial source, the heteroaromatics where B is -COOH are subjected to homologation by successive treatment under Arndt-Eistert conditions. These acids are then esterified by the general procedure outlined in the preceeding paragraph.

To effect the coupling of the thiochroman moiety with those of formula III, the halo-substituted heteroaromatic compound is dissolved in a dry reaction solvent. The heteromatic compound is used in an amount approximating the molar concentration of formula 7. This solution is introduced into a suspension of tetrakis-triphenylphosphine palladium (about a 5 to 10% molar amount relative to the reactants) in the reaction solvent at a temperature of between about -10° and +10°C. This mixture is stirred briefly, for about 15 minutes. To this just-prepared mixture is then added the pre-prepared solution of formula 7, the addition being made at about room temperature. This solution is stirred for an extended period, between about 15 and 25 hours at room temperature. The reaction is then quenched with acid and the product separated and purified by conventional means to give the compounds of formula I.

An alternative means for making compounds where n is 1 - 5 is to subject the compounds of formula I where B is an acid function to homologation using the Arndt-Eistert method referred to above.

The acids and salts derived from formula I are readily obtainable from the corresponding esters. Basic saponification with an alkali metal base will provide the acid. For example, an ester of formula I may be dissolved in a polar solvent such as an alkanol, preferably under an inert atmosphere at room temperature, with about a three molar excess of base, for example, potassium hydroxide. The solution is stirred for an extended period of time, between 15 and 20 hours, cooled, acidified and the hydrolysate recovered by conventional means.

The amide may be formed by any appropriate amidation means known in the art. One way to prepare such compounds is to convert an acid to an acid chloride and then treat that compound with ammonium hydroxide or an appropriate amine. For example, the acid is treated with an alcoholic base solution such as ethanolic KOH (in approximately a 10% molar excess) at room temperature for about 30 minutes. The solvent is removed and the residue taken up in an organic solvent such as diethyl ether, treated with a dialkyl formamide and then a 10-fold excess of oxalyl chloride. This is all effected at a moderately reduced temperature between about -10° and +10°C. The last mentioned solution is then stirred at the reduced temperature for 1-4 hours, preferably 2 hours. Solvent removal provides a residue which is taken up in an inert inorganic solvent such as benzene, cooled to about 0°C and treated with concentrated ammonium hydroxide. The resulting mixture is stirred at a reduced temperature for 1-4 hours. The product is recovered by conventional means.

Alcohols are made by converting the corresponding acids to the acid chloride with thionyl chloride or other means (J. March, "Advanced Organic Chemistry", 2nd Edition, McGraw-Hill Book Company) then reducing the acid chloride with sodium borohydride (March, Ibid, pg. 1124), which gives the corresponding alcohols. Alternatively, esters may be reduced with lithium aluminum hydride at reduced temperatures. Alkylating these alcohols with appropriate alkyl halides under Williamson reaction conditions (March, Ibid, pg. 357) gives the corresponding ethers. These alcohols can be converted to esters by reacting them with appropriate acids in the presence of acid catalysts or dicyclohexylcarbodiimide and dimethylaminopyridine.

Aldehydes can be prepared from the corresponding primary alcohols using mild oxidizing agents such as pyridinium dichromate in methylene chloride (Corey, E.J., Schmidt, G., Tet. Lett., 399, 1979), or dimethyl sulfoxide/oxalyl chloride in methylene chloride (Omura, K., Swern, D. Tetrahedron, 1978, 34, 1651).

Ketones can be prepared from an appropriate aldehyde by treating the aldehyde with an alkyl Grignard reagent or similar reagent followed by oxidation.

Acetals or ketals can be prepared from the corresponding aldehyde or ketone by the method described in March, Ibid, p 810.

Compounds where B is H are prepared from the corresponding halo-heterocyclic entity preferably where the halogen is I. This haloheterocyclic compound is reacted with the ethynyl zinc chloride entity as described in Reaction Scheme I and more specifically in Example 6. Halo-substituted heterocyclic compounds where B is H are commercially available or can be prepared by methods in the literature.

Compounds where X is oxygen are prepared by the steps outlined in Reaction Scheme II. The phosphate of formula 10 is prepared from the corresponding diphenyl chlorophosphate and 3-methyl-3-butene-1-ol available from Aldrich or which may be prepared by means known in the art. It is preferred to prepare formula 10 by dissolving the alcohol of formula 9 in about a 10% excess of pyridine in a polar inert solvent under an inert atmosphere cooled to approximately -10 to 10°C. This solution is then added drop-wise, under an inert atmosphere, to a solution of cooled diphenyl chlorophosphate in about an equal amount of the reaction solvent. About a 2-5% molar excess of diphenyl chlorophosphate relative to the alcohol is employed. The atmosphere may be argon, nitrogen, or another inert gas. The mixture is heated at reflux for between 1 and 5 hours, preferably about 3, to effect the reaction. The product is then recovered by conventional means.

The diphenyl phosphate ester from the preceding paragraph (formula 10) is then reacted with phenol or 3-methylphenol to effect formation of compound 11. For example, phenol is added to a flask already containing stannic chloride under argon which has been cooled to between -10 to 10°C. After thorough mixing of this combination for about 15 minutes to an hour at the reduced temperature, the phosphate is added at the reduced temperature. Both of these steps are carried out under an inert atmosphere such as argon or nitrogen. When the addition of the phosphate is completed, the mixture is stirred at about ambient temperature for up to 24 hours. Then the reaction is quenched with a dilute solution of aqueous alkali metal base. The product is recovered by extraction and other conventional means.

Formula 11 is then acetylated, converted to the acetylene and then to the alkynyl zinc chloride salt and thereafter coupled with the appropriate heterocycle by the steps outlined in Reaction Scheme I.

The tetrahydroquinoline moiety, that is where X is nitrogen, is made in part by the method described in European Patent Application 0130795 published September 1, 1985. First, 3-methylcrotonoyl chloride is reacted with aniline to obtain the amide. This amide is then cyclized using aluminum chloride in the absence of solvent. Lithium aluminum hydride or another acceptable reducing agent of similar type is then used to reduce the 2-oxo-1,2,3,4-tetrahydroquinoline, preferably in an inert solvent such a diethyl ether. This amine is then acetylated using acetyl chloride in a polar solvent such a pyridine. This protected amine is then acetylated in the presence of aluminum chloride. The acetyl function on the nitrogen may then be removed by base hydrolysis. Then the acetylated compound is converted to the acetylene and ZnCl salt as outlined in Reaction Scheme I. This salt is then coupled with an appropriate compound of formula III as described before to give compounds of formula I.

The following Examples are set out to illustrate the the invention, not to limit its scope.

### EXAMPLE 1

### Phenyl-3-methylbut-2-enylsulfide

A mixture of 14.91g (135.324 mmol) of thiophenol and 5.5g (137.5 mmol) of NaOH in 100 ml acetone was heated at reflux for 2.5 hours and then treated dropwise with a solution of 20g (134.19 mmol) of 1-bromo-3-methyl-2-butene in 20ml acetone. This solution was refluxed for 40 hours and then stirred at room temperature for 24 hours. Solvent was then removed in vacuo, the residue taken up in water, and extracted with 3x50 ml ether. Ether extracts were combined and washed with 3x30 ml of 5% NaOH solution, then water, saturated NaCl solution and dried (MgSO₄). Solvent was then removed in vacuo and the residue further purified by kugelrohr distillation (80°C, 0.75 mm) to give the title compound as a pale yellow oil. PMR (CDCl₃) :δ1.57 (3H, s), 1.69 (3H, s), 3.52 (2H, d, J∼7.7Hz), 5.29 (1H, t, J∼7.7Hz), 7.14 (1H, t, J∼7.0 Hz), 7.24 (2H, t, J∼7.0 Hz), 7.32 (2H, d, J∼7.0 Hz).

### EXAMPLE 2

### 4,4-Dimethylthiochroman

To a solution of 15.48g (86.824 mmol) of phenyl-3-methylbut-2-enylsulfide (from Example 1) in 160 ml benzene were added successively 12.6 g (88.767 mmol) of phosphorus pentoxide and 11 ml of 85% phosphoric acid. This solution was refluxed with vigorous stirring under argon for 20 hours, then cooled to room temperature. The supernatant organic layer was decanted and the syrupy residue extracted with 3x50ml ether. Organic fractions were combined and washed with water, saturated NaHCO₃ and saturated NaCl solution and then dried (MgSO₄). Solvent was removed in vacuo and the residue purified by kugelrohr distillation (80°C, 0.5mm) to give the title compound as a pale yellow oil. PMR (CDCl₃) :δ 1.30 (6H, s), 1.90-1.95 (2H, m), 2.97-3.00 (2H, m), 6.96-7.00 (2H, m), 7.04-7.07 (1H, m), 7.30-7.33 (1H, m).

This method can be used to make 6-position alkyl analogues as exemplified by the following compounds:
4,4,7-trimethylthiochroman;
4,4-dimethyl-7-ethylthiochroman;
4,4-dimethyl-7-propylthiochroman;
4,4-dimethyl-7-butylthiochroman; and
4,4-dimethyl-7-hexylthiochroman.

### EXAMPLE 3

### 4,4 Dimethyl-6-acetylthiochroman

A solution of 14.3 g (80.21 mmol) of 4,4-dimethyl thiochroman (from Example 2) and 6.76 g (86.12 mmol) of acetyl chloride in 65 ml benzene was cooled in an ice bath and treated dropwise with 26.712 g (102.54 mmol) of stannic chloride. The mixture was stirred at room temperature for 12 hours, then treated with 65ml water and 33ml conc. hydrogen chloride and heated at reflux for 0.5 hours. After being cooled to room temperature, the organic layer was separated and the aqueous layer extracted with 5x50ml benzene. The recovered organic fractions were combined and washed with 5% sodium carbonate solution, water, saturated NaCl solution and then dried (MgSO₄). The solvent was removed in vacuo and the residue purified by flash chromatography (silica; 5% ethyl acetate in hexanes) followed by kugelrohr distillation (150°C, 0.7mm) to give the title compound as a pale yellow oil. PMR (CDCl₃): δ 1.35 (6H, s), 1.92-1.98 (2H, m) 2.54 (3H, s), 3.02-3.08 (2H, m), 7.13 (1H, d, J∼8.6 Hz), 7.58 (1H, dd, J∼8.6 Hz, 2Hz), 7.99 (1H, d, J∼2Hz).

This same method may be used to form the 6-acetyl compound from those made as per per Example 2.

### EXAMPLE 4

### 4,4-Dimethyl-6-ethynylthiochrman

To a solution of 1.441g (14.2405 mmol) of diisopropylamine in 30ml dry tetrahydrofuran under argon at -78°C was added dropwise 9ml of 1.6M (14.4 mmol) n-butyllithium in hexane. After stirring this solution at -78°C for 1 hour, it was treated dropwise with a solution of 2.95g (13.389 mmol) of 4,4-dimethyl-6-acetylthiochroman in 5ml of dry tetrahydrofuran. After another hour of stirring at -78°C, the solution was treated with 2.507g (14.53mmol) of diethyl chlorophosphate and brought to room temperature, where it was stirred for 3.75 hours. This solution was then transferred using a double ended needle to a solution of lithium diisopropylamide [prepared as above using 2.882g (28.481mmol) of diisopropylamine and 18ml of 1.6M (28.8 mmol) n-butyllithium in hexane] in 60ml dry tetrahydrofuran at -78°C. The cooling bath was removed and the solution stirred at room temperature for 15 hours, then quenched with water and acidified to pH 1 with 3N hydrogen chloride. The mixture was stirred at room temperature for 12 hours, then treated with 65ml water and 33ml conc. hydrogen chloride and heated at reflux for 0.5 hours. After being cooled to room temperature, the organic layer was separated and the aqueous layer extracted with 5x50ml benzene. The recovered organic fractions were combined and washed with 5% sodium carbonate solution, water, saturated NaCl solution and then dried (MgSO₄). The solvent was removed in vacuo and the residue purified by flash chromatography (silica; 5% ethyl acetate in hexanes) followed by kugelrohr distillation (150°C, 0.7mm) to give the captioned compound as a pale yellow oil. PMR (CDCl₃): δ 1.35 (6H, s) 1.92-1.98 (2H, m) 2.54 (3H, s), 3.02-3.08 (2H, m), 7.13 (1H, d, J∼8.6 Hz), 7.58 (1H, dd, J∼8.6 Hz, 2Hz), 7.99 (1H, d, J∼2Hz).

In the same manner, all acetyl-containing prepared under Example 3 may be converted to their corresponding ethynyl analogues.

### EXAMPLE 5

### Ethyl 6-chloronicotinoate

A mixture of 15.75g (0.1 mol) 6-chloronicotinic acid, 6.9g (0.15 mol) ethanol, 22.7g (0.11mol) dicyclohexylcarbodiimide and 3.7 g dimethylaminopyridine in 200 ml methylene chloride was heated at reflux for 2 hours. The mixture was allowed to cool, solvent removed in vacuo and residue subjected to flash chromatography to give the title compound as a low-melting white solid. PMR (CDCl₃): δ 1.44 (3H, t, J∼6.2 Hz) 4.44 (2H, q, J∼4.4 Hz), 7.44 (1H, d, J∼8.1 Hz), 8.27 (1H, dd, J∼8.1 Hz, 3Hz), 9.02 (1H, d, J3Hz).

This procedure may be used to esterify any of the other halo-substituted acids employed in the making of these compounds such as
ethyl 2-(2-chloropyrid-5-yl)acetate;
ethyl 5-(2-chloropyrid-5-yl)pentanoate;
ethyl 2-(2-iodofur-5-yl)acetate;
ethyl 5-(2-iodofur-5-yl)pentanoate;
ethyl 2-(2-iodothien-5-yl)acetate;
ethyl 5-(2-iodothien-5-yl)pentanoate;
ethyl 2-(3-chloropyridazin-6-yl)acetate;
ethyl 5-(3-chloropyridazin-6-yl)pentanoate; and the corresponding chloro, or other halo, substituted pyrimidinyl or pyrazinyl analogues of such esters.

### EXAMPLE 6

### Ethyl 6-[2-(4,4-dimethylthiochroman-6-yl)ethynyl]nicotinoate

Reaction vessels used in this procedure were flame dried under vacuum and all operations carried out in an oxygen-free, argon or nitrogen atmosphere. To a solution of 465.7 mg (2.3019 mmol) of 4,4-dimethyl-6-ethynylthiochroman in 4 ml of dry tetrahydrofuran at 0°C was added dropwise 1.5 ml of 1.6M (2.4 mmol) n-butyllithium in hexane. This was stirred at 0°C for 10 minutes and at room temperature for 10 minutes, cooled again to 0°C and then treated with a solution of 330 mg (2.4215 mmol) of fused ZnCl₂ in 4ml dry tetrahydrofuran using a double ended needle. Thereafter the solution was stirred at 0°C for 30 minutes, then at room temperature for 10 minutes. A solution of 426.3 mg (2.2967 mmol) of ethyl 6-chloronicotinoate (from Example 5) in 4 ml dry tetrahydrofuran was transferred by double ended needle into a suspension of 430 mg (0.37 mmol) of tetrakistriphenylphosphine palladium in 4 ml dry tetrahydrofuran and stirred at room temperature for 10 minutes, then treated by double ended needle with the solution of the alkynylzinc prepared above. This mixture was stirred at room temperature for 18 hours, then quenched with 100 ml water. Product was recovered by extraction with 3x75ml ether. Ether fractions were combined and washed with saturated NaCl solutions and dried (MgSO₄). Solvent was removed in vacuo and the residue purified by flash chromatography (silica; 5% ethyl acetate in hexane) followed by HPLC (Whatman Partisil M-9 10/50; 4% ethyl acetate in hexane) to give the title compound as a white solid. PMR (CDCl₃): δ 1.36 (6H, s), 1.45 (3H, t, J∼7 Hz), 1.96-2.00 (2H, m), 3.05-3.09 (2H, m), 4.45 (2H, q, J∼7Hz), 7.11 (1H, d, J∼8.4 Hz), 7.29 (1H, dd, J∼8.4Hz, 2.2Hz), 7.59 (1H, d, J∼ 7.8Hz), 7.66 (1H, d, J∼2.2Hz), 8.30 (1H, dd, J∼7.8Hz, 2.3Hz), 9.22 (1H, d, J∼ 2.3 Hz).

Using this method, but substituting the appropriate ethynylthiochroman from Example 4 and the appropriate halo-substituted heteroaromatic ester from Example 5, the following compounds may be prepared:
ethyl 6-[2-(4,4,7-trimethylthiochroman-6-yl)ethynyl]nicotinoate;
ethyl 6-[2-(4,4-dimethyl-7-ethylthiochroman-6-yl)ethynyl]nicotinoate;
ethyl 6-[2-4,4-dimethyl-7-propylthiochroman-6-yl)ethynyl]nicotinoate;
ethyl 6-[2-(4,4-dimethyl-7-hexylthiochroman-6-yl)ethynyl]nicotinoate;
ethyl [2-((4,4-dimethylthiochroman-6-yl)ethynyl)pyrid-5-yl]acetate;
ethyl [2-((4,4,7-trimethylthiochroman-6-yl)ethynyl)pyrid-5-yl]acetate;
ethyl [2-((4,4-dimethyl-7-ethylthiochroman-6-yl)ethynyl)pyrid-5-yl]acetate;
ethyl [2-((4,4-dimethyl-7-hexylthiochroman-6-yl)ethynyl)pyrid-5-yl]acetate;
ethyl 3-[2-((4,4-dimethylthiochrom-2-yl)ethynyl)pyrid-5-yl]propionate;
ethyl 3-[2-((4,4,7-trimethylthiochroman-6-yl)ethynyl)pyrid-5-yl]propionate;
ethyl 3-[2-((4,4-dimethyl-7-ethylthiochroman-6-yl)ethynyl)pyrid-5-yl]propionate;
ethyl 3-[2-((4,4-dimethyl-7-hexylthiochroman-6-yl)ethynyl)pyrid-5-yl]propionate;
ethyl 5-[2-((4,4-dimethylthiochroman-6-yl)ethynyl)pyrid-5-yl]pentanoate;
ethyl 5-[2-((4,4,7-trimethylthiochroman-6-yl)ethynyl)pyrid-5-yl]pentanoate;
ethyl 5-[2-((4,4-dimethyl-7-ethylthiochroman-6-yl)ethynyl)pyrid-5-yl]pentanoate;
ethyl 5-[2-((4,4-dimethyl-7-hexylthiochroman-6-yl)ethynyl)pyrid-5-yl]pentanoate;
ethyl [5-((4,4-dimethylthiochroman-6-yl)ethynyl)fur-2-yl]acetate;
ethyl [5-((4,4,7-trimethylthiochroman-6-yl)ethynyl)fur-2-yl]acetate;
ethyl [5-((4,4-dimethyl-7-ethylthiochroman-6-yl)ethynyl)fur-2-yl]acetate;
ethyl [5-((4,4-dimethyl-7-hexylthiochroman-6-yl)ethynyl)fur-2-yl]acetate;
ethyl 5-[5-((4,4-dimethylthiochroman-6-yl)ethynyl)fur-2-yl]pentanoate;
ethyl 5-[5-((4,4,7-trimethylthiochroman-6-yl)ethynyl)fur-2-yl]pentanoate;
ethyl 5-[5-((4,4-dimethyl-7-ethylthiochroman-6-yl)ethynyl)fur-2-yl]pentanoate;
ethyl 5-[5-((4,4-dimethyl-7-hexylthiochroman-6-yl)ethynyl)fur-2-yl]pentanoate;
ethyl [5-((4,4-dimethylthiochroman-6-yl)ethynyl)thien-2-yl]acetate;
ethyl [5-((4,4,7-trimethylthiochroman-6-yl)ethynyl)thien-2-yl]acetate;
ethyl [5-((4,4-dimethyl-7-ethylthiochroman-6-yl)ethynyl)thien-2-yl]acetate;
ethyl [5-((4,4-dimethyl-7-hexylthiochroman-6-yl)ethynyl)thien-2-yl]acetate;
ethyl 5-[5-((4,4-dimethylthiochroman-6-yl)ethynyl)thien-2-yl]pentanoate;
ethyl 5-[5-((4,4,7-trimethylthiochroman-6-yl)-ethynyl)thien-2-yl]pentanoate;
ethyl 5-[5-((4,4-dimethyl-7-ethylthiochroman-6-yl)ethynyl)thien-2-yl]pentanoate;
ethyl 5-[5-((4,4-dimethyl-7-hexylthiochroman-6-yl)ethynyl)thien-2-yl]pentanoate;
ethyl [6-((4,4-dimethylthiochroman-6-yl)ethynyl)pyridazin-3-yl]acetate;
ethyl [6-((4,4,7-trimethylthiochroman-6-yl)ethynyl)pyridazin-3-yl]acetate;
ethyl [6-((4,4-dimethyl-7-ethylthiochroman-6-yl)ethynyl)pyridazin-3-yl]acetate;
ethyl [6-((4,4-dimethyl-7-hexylthiochroman-6-yl)ethynyl)pyridazin-3-yl]acetate;
ethyl 5-[6-((4,4-dimethylthiochroman-6-yl)ethynyl)pyridazin-3-yl]pentanoate;
ethyl 5-[6-((4,4,7-trimethylthiochroman-6-yl)ethynyl)pyridazin-3-yl]pentanoate;
ethyl 5-[6-((4,4-dimethyl-7-ethylthiochroman-6-yl)ethynyl)pyridazin-3-yl]pentanoate;
ethyl 5-[6-((4,4-dimethyl-7-hexylthiochroman-6-yl)ethynyl)pyridazin-3-yl]pentanoate;
ethyl [5-((4,4-dimethylthiochroman-6-yl)ethynyl)pyrimidin-2-yl]acetate;
ethyl [5-((4,4,7-trimethylthiochroman-6-yl)ethynyl)pyrimidin-2-yl]acetate;
ethyl [5-((4,4-dimethyl-7-ethylthiochroman-6-yl)ethynyl)pyrimidin-2-yl]acetate;
ethyl [5-((4,4-dimethyl-7-hexylthiochroman-6-yl)ethynyl)pyrimidin-2-yl]acetate;
ethyl 5-[5-((4,4-dimethylthiochroman-6-yl)ethynyl)pyrimidin-2-yl]pentanoate;
ethyl 5-[5-((4,4,7-trimethylthiochroman-6-yl)ethynyl)pyrimidin-2-yl]pentanoate;
ethyl 5-[5-((4,4-dimethyl-7-ethylthiochroman-6-yl)ethynyl)pyrimidin-2-yl]pentanoate;
ethyl 5-[5-((4,4-dimethyl-7-hexylthiochroman-6-yl)ethynyl)pyrimidin-2-yl]pentanoate;
ethyl [5-((4,4-dimethylthiochroman-6-yl)ethynyl)pyrazin-2-yl]acetate;
ethyl [5-((4,4,7-trimethylthiochroman-6-yl)ethynyl)pyrazin-2-yl]acetate;
ethyl [5-((4,4-dimethyl-7-ethylthiochroman-6-yl)ethynyl)pyrazin-2-yl]acetate;
ethyl [5-((4,4-dimethyl-7-hexylthiochroman-6-yl)ethynyl)pyrazin-2-yl]acetate;
ethyl 5-[5-((4,4-dimethylthiochroman-6-yl)ethynyl)pyrazin-2-yl]pentanoate;
ethyl 5-[5-((4,4,7-trimethylthiochroman-6-yl)ethynyl)pyrazin-2-yl]pentanoate;
ethyl 5-[5-((4,4-dimethyl-7-ethylthiochroman-6-yl)ethynyl)pyrazin-2-yl]pentanoate; and
ethyl 5-[5-((4,4-dimethyl-7-hexylthiochroman-6-yl)ethynyl)pyrazin-2-yl]pentanoate.

### EXAMPLE 7

### Diphenyl-3-methyl-3-buten-1-yl phosphate

To an ice-cooled solution of 12.2g (141.65 mmol) of 3-methyl-3-buten-1-ol (Aldrich) and 11.9g (150.44 mmol) of pyridine in 100ml of tetrahydrofuran was added dropwise under argon a solution of 38.5g (143.21 mmol) of diphenyl chlorophosphate 93 in 100ml of tetrahydrofuran. The mixture was heated at reflux for 3 hours and then cooled and filtered. The filtrate was concentrated in vacuo and the residue dissolved in 400ml of 1:1 ether and hexane and then washed with 2x200ml water, 75ml saturated NaCl solution and dried (MgSO₄). The solvent was removed in vacuo to give the captioned compound as a pale yellow oil. PMR (CDCl₃): δ1.69 (3H, ∂), 2.37 (2H, t, J∼7Hz), 4.32 (2H, q, J∼7Hz), 4.72 (1H, ∂), 4.80 (1H, ∂), 7.10-7.35 (10H, m).

### EXAMPLE 8

### 4,4-Dimethylchroman

To a dry, ice-cooled flask containing 34.95g (0.134 mol) of stannic chloride was added quickly under argon 63.0g (0.669 mol) of phenol. The mixture was stirred at 0°C for 0.5 hour and then treated with 43.0g (0.135 mol) of diphenyl-3-methyl-3-buten-1-yl phosphate, followed by a 5ml carbon disulfide rinse. The mixture was stirred at room temperature for 21 hours and then quenched by pouring onto 700g ice and 1 litre of 1.5N NaOH. The mixture was extracted with 1x600ml and 2x300 ml ether. The combined ether fractions were washed with 2N NaOH, saturated NaCl and dried (MgSO₄). Solvent was removed in vacuo and the residue purified by flash chromatography (silica; 2% ether in hexane) to give the title compound as a colorless oil. PMR (CDCl₃)δ: 1.34 (6H, ∂), 1.80-1.85 (2H, m), 4.15-4.20 (2H, m), 6.80 (1H, dd, J∼8.1Hz, 1.5Hz), 6.87 (1H, td, J∼8.1Hz, 1.5 Hz), 7.07 (1H, td, J∼8.1Hz, 1.5Hz), 7.26 (1H, dd, J∼8.1Hz, 1.5Hz).

This method will serve to prepare the corresponding 7-alkylchroman compounds, starting with the appropriate 3-alkylphenol:
4,4,7-trimethylchroman;
4,4-dimethyl-7-ethylchroman;
4,4-dimethyl-7-propylchroman;
4,4-dimethyl-7-butylchroman;
4,4-dimethyl-7-pentylchroman; and
4,4-dimethyl-7-hexylchroman.

### EXAMPLE 9

### 4,4-dimethyl-6-acetylchroman

To a stirred solution of 7.94g (48.9425 mmol) of 4,4-dimethylchroman in 70ml of nitromethane was added under argon 4.0g (50.96 mmol) of acetyl chloride followed by 6.8g (51 mmol) of aluminum chloride. This was stirred at room temperature for 5.5 hours and then cooled in an ice bath and treated slowly with 70ml 6N hydrogen chloride. The resultant mixture was stirred at room temperature for 10 minutes, then treated with 100ml ether and the organic layer separated. The organic layer was washed with water, saturated NaHCO₃ and saturated NaCl solutions and dried (MgSO₄). Solvent was removed in vacuo and the residue purified by flash chromatography (silica; 10% ethyl acetate in hexanes). This was followed by kugelrohr distillation (95-100°C; 0.15 mm) to give the title compound as a colorless oil. PMR (CDCl₃): δ 1.40 (6H, ∂), 1.95-2.00 (2H, m) 2.58 (3H, ∂), 4.25-4.30 (2H, m), 6.83 (1H, d, J∼8.0Hz), 7.62 (1H, dd, J∼8.0Hz, 1.5Hz), 8.00 (1H, d, J∼1.5Hz).

Following the same procedure and using the compounds of Example 8, the following compounds can be prepared:
4,4-dimethyl-6-acetyl-7-methylchroman;
4,4-dimethyl-6-acetyl-7-ethylchroman;
4,4-dimethyl-6-acetyl-7-propylchroman;
4,4-dimethyl-6-acetyl-7-butylchroman;
4,4-dimethyl-6-acetyl-7-pentylchroman; and
4,4-dimethyl-6-acetyl-7-hexylchroman.

### EXAMPLE 10

### 4,4-Dimethyl-6-ethynylchroman

To a solution of 2.47g (24.41mmol) of diisopropylamine in 40ml dry tetrahydrofuran under argon at -78°C was added dropwise 15.2ml of 1.6M (24.32 mmol) n-butyllithium in hexane. Mixture was stirred at -78°C for 1 hour and then treated dropwise with a solution of 4.98g (24.38 mmol) of 4,4-dimethyl-6-acetylchroman in 4ml dry of tetrahydrofuran. After stirring at -78°C for 1 hour, the solution was treated with 4.2g (24.36 mmol) of diethyl chlorophosphate. The cooling bath was then removed and mixture stirred at room temperature for 2.75 hours. This solution was then transferred using a double ended needle to a solution of lithium diisopropyl amide (prepared as per Example 4 using 4.95g (48.92 mmol) of diisopropylamine and 30.5 ml of 1.6M (48.8 mmol) n-butyllithium in hexane in 80ml dry tetrahydrofuran at -78°C. The cooling bath was removed and mixture stirred at room temperature for 18 hours and then quenched with 50ml water and 25ml of 3N hydrogen chloride. The mixture was extracted with 2x100ml and 3x50ml of pentane and the combined organic fractions washed with 3N hydrogen chloride, water, saturated NaHCO₃ and saturated NaCl solution and then dried (MgSO₄). Solvent was then removed in vacuo and the residue purified by flash chromatography (silica; 10% ethyl acetate in hexane) followed by kugelrohr distillation (70°C; 0.35mm) to give the title compound as a colorless crystalline solid. PMR (CDCl₃): δ 1.33 (6H, s), 1.81-1.86 (2H, m), 3.00 (1H, s), 4.19-4.24 (2H, m), 6.75 (1H, d, J∼8.5Hz), 7.22 (1H, dd, J∼8.5 Hz, 2.3Hz), 7.44 (1H, d, J∼2.3Hz).

This procedure serves to convert all 6-acetyl compounds prepared as per Example 9 to their corresponding 6-ethynyl analogues.

### EXAMPLE 11

### Ethyl 6-[2-(4,4-dimethylchroman-6-yl)ethynyl]nicotinoate

Reaction vessels used in this procedure were flame dried under vacuum and all operations were carried out in an oxygen-free, argon or nitrogen atmosphere. To a solution of 509.4mg (2.74 mmol) of 4,4-dimethyl-6-ethynyl chroman in 4ml of dry tetrahydrofuran at 0°C was added dropwise 1.72ml of 1.6M (2.75 mmol) of n-butyllithium in hexane. Stirring was commenced at 0°C for 30 minutes and at room temperature for 15 minutes, after which the solution was cooled again to 0°C and then treated with a solution of 380mg (2.79 mmol) of fused zinc chloride in 5ml of dry tetrahydrofuran using a double ended needle. The resulting solution was stirred at 0°C for 1 hour and then at room temperature for 15 minutes. A solution of 628.6mg (2.74 mmol) of ethyl 6-chloronicotinoate in 4ml of dry tetrahydrofuran was transferred by double ended needle into a suspension of 380mg (0.33 mmol) of tetrakistriphenylphosphine palladium in 5ml dry tetrahydrofuran and mixture stirred at room temperature for 15 minutes and then treated by double ended needle with the solution of alkynylzinc prepared above. The mixture was stirred at room temperature for 20 hours and then quenched with ice and 30ml of 3N hydrogen chloride. The mixture was extracted with 3x75ml ether and ether extracts were combined and washed successively with saturated NaHCO₃ and saturated NaCl and then dried (MgSO₄). Solvent was removed in vacuo and the residue further purified by flash chromatography (silica; 10% ethyl acetate in hexane) to give the title compound as a yellow solid. PMR (CDCl₃): δ 1.36 (6H, s), 1.44 (3H, t, J∼7.1Hz), 1.83-1.87 (2H, m), 4.22-4.26 (2H, m), 4.44 (2H, q, J∼7.1Hz), 6.80 (1H, d, J∼7.6 Hz), 7.35 (1H, d, 7∼8.9Hz), 7.58 (1H, d, J∼7.6 Hz), 7.60 (1H, ∂), 8.28 (1H, d, J∼8.9Hz), 9.21 (1H, s).

By this method, using the appropriate precursors, the following compounds are prepared:
ethyl 6-[2-(4,4,7-trimethylchroman-6-yl)ethynyl]nicotinoate;
ethyl 6-[2-(4,4-dimethyl-7-ethylchroman-6-yl)ethynyl]nicotinoate;
ethyl 6-[2-(4,4-dimethyl-7-propylchroman-6-yl)ethynyl]nicotinoate;
ethyl 6-[2-(4,4-dimethyl-7-hexylchroman-6-yl)ethynyl]nicotinoate;
ethyl [2-((4,4-dimethylchroman-6-yl)ethynyl)pyrid-5-yl]acetate;
ethyl [2-((4,4,7-trimethylchroman-6-yl)ethynyl)pyrid-5-yl]acetate;
ethyl [2-((4,4-dimethyl-7-ethylchroman-6-yl)ethynyl)pyrid-5-yl]acetate;
ethyl [2-((4,4-dimethyl-7-hexylchroman-6-yl)ethynyl)pyrid-5-yl]acetate;
ethyl 3-[2-((4,4-dimethylchroman-2-yl)ethynyl)pyrid-5-yl]propionate;
ethyl 3-[2-((4,4,7-trimethylchroman-6-yl)ethynyl)pyrid-5-yl]propionate;
ethyl 3-[2-((4,4-dimethyl-7-ethylchroman-6-yl)ethynyl)pyrid-5-yl]propionate;
ethyl 3-[2-((4,4-dimethyl-7-hexylchroman-6-yl)ethynyl)pyrid-5-yl]propionate;
ethyl 5-[2-((4,4-dimethylchroman-6-yl)ethynyl)pyrid-5-yl]pentanoate;
ethyl 5-[2-((4,4,7-trimethylchroman-6-yl)ethynyl)pyrid-5-yl]pentanoate;
ethyl 5-[2-((4,4-dimethyl-7-ethylchroman-6-yl)ethynyl)pyrid-5-yl]pentanoate;
ethyl 5-[2-((4,4-dimethyl-7-hexylchroman-6-yl)ethynyl)pyrid-5-yl]pentanoate;
ethyl [5-((4,4-dimethylchroman-6-yl)ethynyl)fur-2-yl]acetate;
ethyl [5-((4,4,7-trimethylchroman-6-yl)ethynyl)fur-2-yl]acetate;
ethyl [5-((4,4-dimethyl-7-ethylchroman-6-yl)ethynyl)fur-2-yl]acetate;
ethyl [5-((4,4-dimethyl-7-hexylchroman-6-yl)ethynyl)fur-2-yl]acetate;
ethyl 5-[5-((4,4-dimethylchroman-6-yl)ethynyl)fur-2-yl]pentanoate;
ethyl 5-[5-((4,4,7-trimethylchroman-6-yl)ethynyl)fur-2-yl]pentanoate;
ethyl 5-[5-((4,4-dimethyl-7-ethylchroman-6-yl)ethynyl)fur-2-yl]pentanoate;
ethyl 5-[5-((4,4-dimethyl-7-hexylchroman-6-yl)ethynyl)fur-2-yl]pentanoate;
ethyl [5-((4,4-dimethylchroman-6-yl)ethynyl)thien-2-yl]acetate;
ethyl [5-((4,4,7-trimethylchroman-6-yl)ethynyl)thien-2-yl]acetate;
ethyl [5-((4,4-dimethyl-7-ethylchroman-6-yl)ethynyl)thien-2-yl]acetate;
ethyl [5-((4,4-dimethyl-7-hexylchroman-6-yl)ethynyl)thien-2-yl]acetate;
ethyl 5-[5-((4,4-dimethylchroman-6-yl)ethynyl)thien-2-yl]pentanoate;
ethyl 5-[5-((4,4,7-trimethylchroman-6-yl)ethynyl)thien-2-yl]pentanoate;
ethyl 5-[5-((4,4-dimethyl-7-ethylchroman-6-yl)ethynyl)thien-2-yl]pentanoate;
ethyl 5-[5-((4,4-dimethyl-7-hexylchroman-6-yl)ethynyl)thien-2-yl]pentanoate;
ethyl [6-((4,4-dimethylchroman-6-yl)ethynyl)pyridazin-3-yl]acetate;
ethyl [6-((4,4,7-trimethylchroman-6-yl)ethynyl)pyridazin-3-yl]acetate;
ethyl [6-((4,4-dimethyl-7-ethylchroman-6-yl)ethynyl)pyridazin-3-yl]acetate;
ethyl [6-((4,4-dimethyl-7-hexylchroman-6-yl)ethynyl)pyridazin-3-yl]acetate;
ethyl 5-[6-((4,4-dimethylchroman-6-yl)ethynyl)pyridazin-3-yl]pentanoate;
ethyl 5-[6-((4,4,7-trimethylchroman-6-yl)ethynyl)pyridazin-3-yl]pentanoate;
ethyl 5-[6-((4,4-dimethyl-7-ethylchroman-6-yl)ethynyl)pyridazin-3-yl]pentanoate;
ethyl 5-[6-((4,4-dimethyl-7-hexylchroman-6-yl)ethynyl)pyridazin-3-yl]pentanoate;
ethyl [5-((4,4-dimethylchroman-6-yl)ethynyl)pyrimidin-2-yl]acetate;
ethyl [5-((4,4,7-trimethylchroman-6-yl)ethynyl)pyrimidin-2-yl]acetate;
ethyl [5-((4,4-dimethyl-7-ethylchroman-6-yl)ethynyl)pyrimidin-2-yl]acetate;
ethyl [5-((4,4-dimethyl-7-hexylchroman-6-yl)ethynyl)pyrimidin-2-yl]acetate;
ethyl 5-[5-((4,4-dimethylchroman-6-yl)ethynyl)pyrimidin-2-yl]pentanoate;
ethyl 5-[5-((4,4,7-trimethylchroman-6-yl)ethynyl)pyrimidin-2-yl]pentanoate;
ethyl 5-[5-((4,4-dimethyl-7-ethylchroman-6-yl)ethynyl)pyrimidin-2-yl]pentanoate;
ethyl 5-[5-((4,4-dimethyl-7-hexylchroman-6-yl)ethynyl)pyrimidin-2-yl]pentanoate;
ethyl [5-((4,4-dimethylchroman-6-yl)ethynyl)pyrazin-2-yl]acetate;
ethyl [5-((4,4,7-trimethylchroman-6-yl)ethynyl)pyrazin-2-yl]acetate;
ethyl [5-((4,4-dimethyl-7-ethylchroman-6-yl)ethynyl)pyrazin-2-yl]acetate;
ethyl [5-((4,4-dimethyl-7-hexylchroman-6-yl)ethynyl)pyrazin-2-yl]acetate;
ethyl 5-[5-((4,4-dimethylchroman-6-yl)ethynyl)pyrazin-2-yl]pentanoate;
ethyl 5-[5-((4,4,7-trimethylchroman-6-yl)ethynyl)pyrazin-2-yl]pentanoate;
ethyl 5-[5-((4,4-dimethyl-7-ethylchroman-6-yl)ethynyl)pyrazin-2-yl]pentanoate; and
ethyl 5-[5-((4,4-dimethyl-7-hexylchroman-6-yl)ethynyl)pyrazin-2-yl]pentanoate.

### EXAMPLE 12

### 6-[2-(4,4-dimethylchroman-6-yl)ethynyl]nicotinic Acid

The absolute ethanol used in this experiment was degassed by applying a vacuum while simultaneously bubbling nitrogen through it. A solution of 101.1mg (0.30 mmol) of ethyl 6-[2-(4,4-dimethylchroman-6-yl)ethynyl]nicotinoate in 2ml ethanol was treated under argon with 0.7 ml of a 1.81M (1.27 mmol) solution of potassium hydroxide in ethanol and water. This mixture was stirred at room temperature for 60 hours and then solvent removed in vacuo. The residue was dissolved in 25 ml of water and extracted with 25ml of ether. The aqueous layer was acidified with glacial acetic acid and extracted with 4x50ml of ether. Ether extracts were combined and washed with water, then saturated NaCl and dried (MgSO₄). Solvent was then removed in vacuo to give the title compound. PMR ((CD₃)₂CO):δ 1.40 (6H, s)1.88-1.92 (2H, m), 4.26-4.30(2H, m), 6.82 (1H, d, J∼8.7Hz), 7.37 (1H, dd, J∼7.6Hz, 2.2Hz), 7.62 (1H, ∂), 7.63 (1H, d, J∼8.7Hz), 8.37 (1H, dd, J∼7.6Hz, 2.2Hz), 9.27 (1H, d, J∼2.2Hz).

Proceeding in the same manner 6-[2-(4,4-dimethylthiochroman-6-yl)ethynyl]nicotinic acid was prepared from ethyl 6-[2-(4,4-dimethylthiochroman-6-yl)ethynyl]nicotinoate. PMR [CDCl₃ (CD₃)₂ CO]:δ 1.37(6H,∂), 1.99 (2H, m), 3.09 (2H, m), 7.10 (1H, d, J∼8.1 Hz), 7.28 (1H, dd, J∼8.1 Hz), 2.1 Hz), 7.64 (1H, dd, J∼7.8 Hz), 1.8 Hz), 7.65 (1H, d, J∼7.8 Hz, 1.5 Hz), 9.24 (1H, m).

Proceeding in about the same manner, the esters prepared as per Examples 6 and 11 may be converted the corresponding acids.

### Example 13

### 2-[2-(4,4-Dimethylchroman-6-yl)ethynyl]-5-hydroxymethylpyridine

A 250 ml 3-necked flask is fitted with a stirrer, a dropping funnel, a nitrogen inlet and a thermometer. In the flask is placed a solution of 379.5 mg (10 mmol) of lithium aluminum hydride in 30 ml of dry diethyl ether. The solution is cooled to -65°C under nitrogen and a solution of 3.2343 g (10 mmol) of ethyl 6-[2-(4,4-dimethylchroman-6-yl)ethynyl]-nicotinoate in 15 ml of dry ether is added dropwise at a rate such that the temperature does not exceed -60°C. The mixture is stirred at -30°C for 1 hour and the excess hydride is then destroyed by the addition of 300 mg (3.4 mmol) of ethyl acetate. The reaction mixture is then hydrolyzed by adding 3 ml of saturated ammonium chloride solution and allowing the temperature to rise to room temperature. The mixture is then filtered and the residue washed with ether. The ether layer is then washed with saturated sodium chloride solution, dried (MgSO₄) and then concentrated in vacuo. The residue is purified by chromatography followed by recrystalliztion to give the title compound.

By the same process, acids or esters of this invention may be converted to their corresponding primary alcohol.

### Example 14

### 2-[2-(4,4-Dimethylchroman-6-yl)ethynyl]-5-acetoxymethylpyridine

A solution of 2.81 g (10 mmol) of 4,4-Dimethyl-6-[2-(5-hydroxymethylpyrid-2-yl)ethynyl]chroman, 600 mg (10 mmol) of glacial acetic acid, 2.06 g (10 mmol) of dicyclohexylcarbodiimide and 460 mg (3.765 mmol) of 4-dimethylaminopyridine in 150 ml methylene chloride is stirred at room temperature for 48 hours. The reaction mixture is then filtered and the residue washed with 50 ml of methylene chloride. The filtrate is then concentrated in vacuo and the residue is purified by chromatography followed by recrystallation to give the title compound.

Proceeding in the same manner, other alcohols of this invention may be esterified.

### Example 15

### 2-[2-(4,4-Dimethylchroman-6-yl)ethynyl]pyridine-5-carboxaldehyde

A solution of 1.396 g (11 mmol) of freshly distilled oxalyl chloride in 25 ml of methylene chloride is placed in a 4-necked flask equipped with a stirrer, a thermometer and two pressure-equalizing addition funnels fitted with drying tubes. The solution is cooled to -60°C and then treated dropwise with a solution of 1.875 g (24 mmol) of dimethyl sulfoxide (distilled from calcium hydride) in 5 ml of methylene chloride over a five minute period. The reaction mixture is then stirred at -60°C for an additional 10 minutes. A solution of 2.81 g (10 mmol) of 4,4-dimethyl-6-[2-(5-hydroxymethylpyrid-2-yl)ethynyl]chroman in 10 ml of methylene chloride is then added to the reaction mixture over a period of 5 minutes. The mixture is stirred for a further 15 minutes and is then treated with 5.06 g (50 mmol) of triethylamine. The cooling bath is then removed and the mixture is allowed to warm to room temperature. Thirty ml of water is then added to the mixture and stirring is continued for a further 10 minutes. The organic layer is then separated and the aqueous layer is extracted with 20 ml of methylene chloride. The organic layers are then combined and washed successively with dilute HCl, water and dilute Na₂CO₃ solution and then dried (MgSO₄). The solution is then filtered and concentrated in vacuo and the residue is purified by chromatography followed by recrystallization to give the title compound.

Primary alcohols of this invention may be oxidized to their corresponding aldehyde by this method.

### Example 16

### 2-[2-(4,4-Dimethylchroman-6-yl)ethynyl]-5-(1-hydroxypropyl)pyridine

Four ml of a 3 M (12 mmol) solution of ethylmagnesium bromide in ether is placed in a 3-necked flask fitted with a mechanical stirrer, a reflux condenser protected by a drying tube and a pressure-equalizing dropping funnel protected by a drying tube. The flask is cooled in an ice-bath nd a solution of 2.8 g (10 mmol) of 2-[2-(4,4-Dimethylchroman-6-yl)ethynyl]-pyridine-5-carboxaldehyde in 10 ml of dry ether is added slowly with vigorous stirring. The cooling bath is then removed and the mixture heated at reflux for 3 hours. The mixture is then cooled in an ice-salt bath and 5 ml of saturated ammonium chloride solution added. The mixture is stirred for a further 1 hour and then filtered and the residue washed with two 10 ml portions of ether. The ether solution is then separated, dried (MgSO₄) and the ether removed in vacuo. The residue is then purified by chromatography followed by recrystallization to give the title compound.

Using the same procedure any of the other aldehydes of this invention can be converted to a secondary alcohol.

Such secondary alcohols may be converted to their corresponding ketone using the procedure recited in Example 15.

### Example 17

### 2-[2-(4,4-Dimethylchroman-6-yl)ethynyl]-5-dimethoxymethylpyridine

A round-bottomed flask is fitted with a Dean-Stark apparatus under a reflux condenser protected by a drying tube. A mixture of 3.35 g (12 mmol) of 2-[2-(4,4-Di-methyl-chroman-6-yl)ethynyl]-pyridine-5-carboxaldehyde, 4.80 mg (15 mmol) of anhydrous methanol, 2 mg of p-toluenesulfonic acid monohydrate and 10 ml of anhydrous benzene is placed in the flask and the mixture heated at reflux under nitrogen until close to the theoretical amount of water is collected in the Dean-Stark trap. The reaction mixture is cooled to room temperature and extracted successively with 5 ml of 10% sodium hydroxide solution and two 5 ml portions of water and then dried (MgSO₄). The solution is then filtered and the solvent removed in vacuo. The residue is purified by chromatography and then recrystalliztion to give the title compound.

In a similar manner, any aldehyde or ketone of this invention may be converted to an acetal or a ketal.

### Example 18

Preferably, these compounds may be administered topically using various formulations. Such formulation may be as follows.

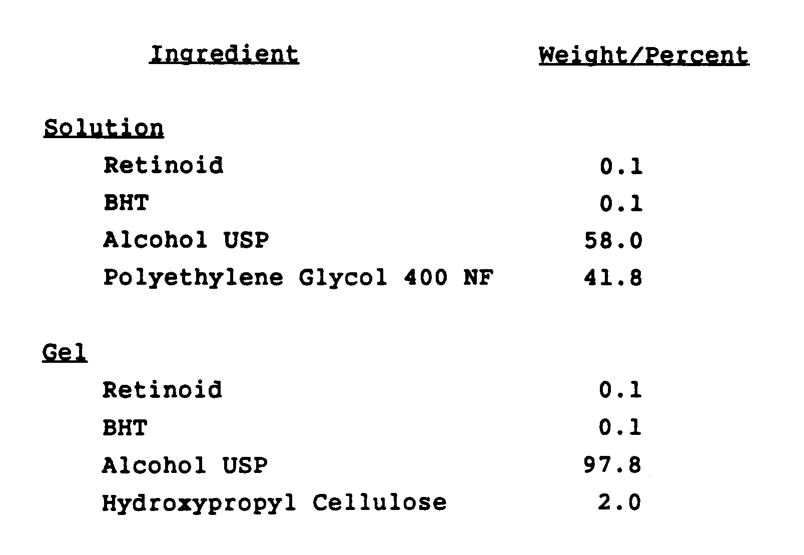

## Claims (Claims for the following Contracting State(s): AT, BE, CH, DE, FR, GB, IT, LI, LU, NL, SE)

1. A compound of the formula I: 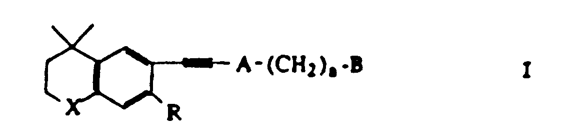 or a salt, alkyl ether, ester or amide thereof, where X is S, O or NR' where R' is hydrogen or C₁₋₆ alkyl; R is hydrogen or C₁₋₆ alkyl; A is pyridyl, thienyl, furyl, pyridazinyl, pyrimidinyl or pyrazinyl; n is 0-5; B is H, CO₂H, CH₂OH, CHO or a C₁₋₆ alkyl acetal derivative thereof, COR₁ or a C₁₋₆ alkyl ketal derivative thereof wherein R₁ is (CH₂)ₘCH₃ and m is 0-4, and wherein the ester is derived from a saturated aliphatic alcohol or acid of up to ten carbon atoms, a cyclic or saturated aliphatic cyclic alcohol or acid of 5 to 10 carbon atoms, or is a phenyl or C₁₋₆ alkylphenyl ester; and the amide is an unsubstituted amide or a mono- or di-substituted amide in which the substituent is a saturated aliphatic radical of up to 10 carbon atoms, or a cyclic or saturated aliphatic-cyclic radical of 5 to 10 carbon atoms or a phenyl or C₁₋₆ alkylphenyl radical.

2. The compound of claim 1 where n is 0.

3. The compound of claim 1 where n is 1, 2, or 3.

4. A compound according to any one of claims 1 to 3 wherein X is S or O, A is pyridyl and B is -COOH, a pharmaceutically acceptable salt thereof, or an ester or amide thereof.

5. A compound of claim 4 which is ethyl 6-[2-(4,4-dimethylthiochroman-6-yl)ethynyl]nicotinoate, 6-[2-(4,4-dimethylthiochromen-6-yl)ethynyl]nicotinic acid, ethyl 6-[2-(4,4-dimethylchroman-6-yl)ethynyl]nicotipoate or 6-[2-(4,4-dimethylchroman-6-yl)ethynyl]nicotinic acid or a pharmaceutically acceptable salt thereof.

6. A compound according to any one of claims 1 to 3 where X is S or O, A is thienyl and B is -COOH or a pharmaceutically acceptable salt, ester or amide thereof.

7. A Compound according to any one of claims 1 to 3 where X is S or O, A is furyl and B is -COOH or a pharmaceutically acceptable salt, ester or amide thereof.

8. A compound according to any one of claims 1 to 3 where X is S or O, A is pyridazinyl, pyrimidinyl or pyrazinyl and B is -COOH or a pharmaceutically acceptable salt, ester or amide thereof.

9. A compound according to any one of claims 1 to 8 for use in medicine.

10. A compound according to any one of claims 1 to 8 for use in treating psoriasis.

11. A pharmaceutical composition comprising a compound defined in any one of claims 1 to 8 and a pharmaceutically acceptable carrier.

12. A process for preparing a compound as defined in any one of claims 1 to 8, which process comprises reacting a compound of the formula II: 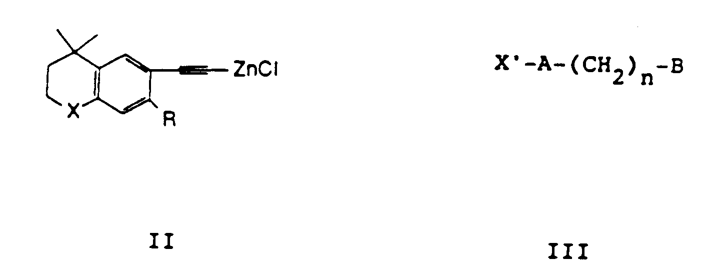 with a compound of the formula III, in the presence of Pd(PQ₃)₄ (Q is phenyl) or a similar complex, wherein X, R, A, and n are as defined in claims 1 to 8, B is a protected acid, alcohol, aldehyde or ketone and X' is a halogen, and thereafter where necessary deprotecting a protected group B and, where desired, converting one compound of the formula I into another compound of the formula I.

13. The use of a compound of the formula I as defined in any one of claims 1 to 8 for the manufacture of a medicament for the treatment of psoriasis.

## Claims (Claims for the following Contracting State(s): ES, GR)

1. A process for preparing a compound of the formula I: 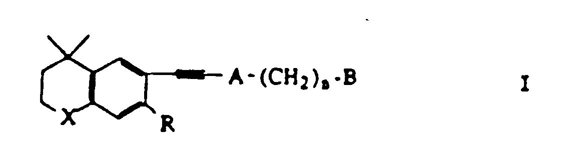 or a salt, alkyl ether, ester or amide thereof, where X is S, O or NR' where R' is hydrogen or C₁₋₆ alkyl; R is hydrogen or C₁₋₆ alkyl; A is pyridyl, thienyl, furyl, pyridazinyl, pyrimidinyl or pyrazinyl; n is 0-5; B is H, CO₂H, CH₂OH, CHO or a C₁₋₆ alkyl acetal derivative thereof, COR₁ or a C₁₋₆ alkyl ketal derivative thereof wherein R₁ is (CH₂)ₘCH₃ and m is 0-4, and wherein the ester is derived from a saturated aliphatic alcohol or acid of up to ten carbon atoms, a cyclic or saturated aliphatic cyclic alcohol or acid of 5 to 10 carbon atoms, or is a phenyl or C₁₋₆ alkylphenyl ester; and the amide is an unsubstituted amide or a mono- or di-substituted amide in which the substituent is a saturated aliphatic radical of up to 10 carbon atoms, or a cyclic or saturated aliphatic-cyclic radical of 5 to 10 carbon atoms or a phenyl or C₁₋₆ alkylphenyl radical; which process comprises reacting a compound of the formula II: 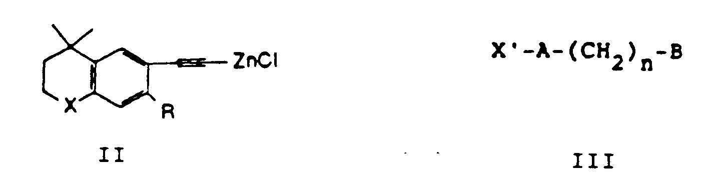 with a compound of the formula (III) in the presence of Pd(PQ₃)₄ (Q is phenyl) or a similar complex, wherein X, R, A and n are as defined above, B is H, or a protected acid, alcohol, aldehyde or ketone, and X' is a halogen, and thereafter where necessary deprotecting a protected group B and where desired, converting one compound of the formula I into another compound of the formula I

2. A process according to claim 1 for preparing a compound where n is O.

3. A process according to claim 1 for preparing a compound where n is 1, 2, or 3.

4. A process according to any one of claims 1 to 3 for preparing a compound wherein X is S or O, A is pyridyl and B is -COOH, a pharmaceutically acceptable salt thereof, or an ester or amide thereof.

5. A process according to claim 4 for preparing a compound which is ethyl 6-[2-(4,4-dimethylthiochroman-6-yl)ethynyl]nicotinoate, 6-[2-(4,4-dimethylthiochroman-6-yl)ethynyl]nicotinic acid, ethyl 6-[2-(4,4-dimethylchroman-6-yl)ethynyl]nicotinoate or 6-[2-(4,4-dimethylchroman-6-yl)ethynyl]nicotinic acid or a pharmaceutically acceptable salt thereof.

6. A process according to any one of claims 1 to 3 for preparing a compound where X is S or O, A is thienyl and B is -COOH or a pharmaceutically acceptable salt, ester or amide thereof.

7. A process according to any one of claims 1 to 3 for preparing a compound where X is S or O, A is furyl and B is -COOH or a pharmaceutically acceptable salt, ester or amide thereof.

8. A process according to any one of claims 1 to 3 for preparing a compound where X is S or O, A is pyridazinyl, pyrimidinyl or pyrazinyl and B is -COOH or a pharmaceutically acceptable salt, ester or amide thereof.

9. A process according to any one of claims 1 to 8 wherein the compound of formula II is reacted with the compound of formula III in the presence of Pd(PQ₃)₄ wherein Q is phenyl.

10. A process for preparing a pharmaceutical composition which comprises bringing a compound as defined in any one of claims 1 to 8 into association with a pharmaceutically acceptable carrier.

11. The use of a compound as defined in any one of claims 1 to 8 in the preparation of a medicament for the treatment of psoriasis.

## Revendications (Revendications pour l'(les) Etat(s) contractant(s) suivant(s): AT, BE, CH, DE, FR, GB, IT, LI, LU, NL, SE)

1. Composé de formule I 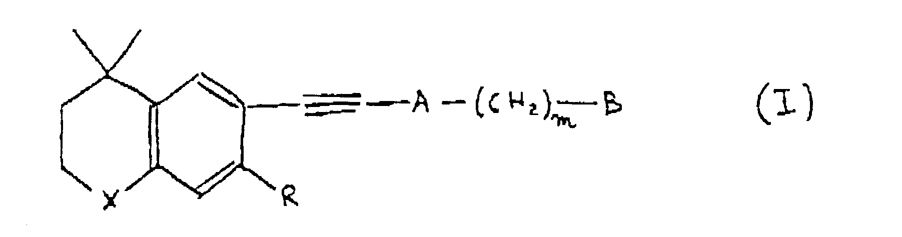 : ou l'un de ses sels, alkyléthers, esters ou amides, formule dans laquelle :
X est S, O ou NR', R' étant l'hydrogène ou on alkyle en C₁ à C₆;
R est l'hydrogène ou un alkyle en C₁ à C₆;
A est un pyridyle, un thiènyle, un furyle, un pyridazinyle, un pyrimidinyle ou un pyrazinyle;
n est de 0 à 5;
B est l'hydrogène, CO₂H, CH₂OH, CHO ou un de leurs dérivés alkyl-acétals en C₁ à C₆, ou COR₁ ou un de ses dérivés alkyl-cétals en C₁ à C₆, dans lequel R1 est (CH₂)ₘCH₃ et m est égal à 0 à 4 :
et dans lequel l'ester est dérivé d'un alcool ou acide aliphatique saturé présentant jusqu'à 10 atomes de carbone, un alcool ou acide cycloaliphatique cyclique ou saturé présentant de 5 à 10 atomes de carbone, ou est un ester phénylique ou phénylique alkylé à 1 à 6 atomes de carbone et dans lequel l'amide est un amide non substitué ou un amide mono ou di-substitué dans lequel le substituant est un radical alipathique saturé présentant jusqu'à 10 atomes de carbone, ou on radical cycloaliphatique saturé ou cyclique présentant de 5 à 10 atomes de carbone ou un radical phénylique ou phénylique alkylé à un à six atomes de carbone,

2. Composé selon la revendication 1 dans lequel n est égal à zéro.

3. Composé selon la revendication 1 dans lequel n est égal à 1,2 ou 3.

4. Composé selon lune quelconque des revendications 1 à 3 dans lequel X est S ou O, A est on pyridyle et B est -COOH, un de ses sels, esters ou amides pharmaceutiquement acceptables.

5. Composé selon la revendication 4 qui est le [(diméthyl-4,4-thiochromanyl-6)-2-acétylényl]-6-nicotinate d'éthyle, l'acide [(diméthyl-4,4-thiochromanyl-6)-2-acétylényl]-6-nicotonique, le (diméthyl-4,4-chromanyl-6)-2-acétylényl]-6-nisotinate d'éthyle et l'acide [(diméthyl-4,4-chromanyl-6)-2-acétylényl]-6-nicotinique ou un sel pharmaceutiquement acceptable de ceux-ci.

6. Composé selon l'une quelconque des revendications 1 à 3 dans lequel X est S ou O, A est un thiényle et B est -COOH ou un de ses sels, esters ou amides, pharmaceutiquement acceptables.

7. Composé selon l'une quelconque des revendications 1 à 3 dans lequel X est S ou O, A est un furyle et B est -COOH ou un de ses sels, esters ou amides, pharmaceutiquement acceptables.

8. Composé selon l'une quelconque des revendications 1 à 3 dans lequel X est S ou O, A est un pyridazinyle, pyrimidinyle ou pyrazinyle et B est -COOH ou un de ses sels, esters ou amides, pharmaceutiquement acceptables.

9. Composé selon l'une quelconque des revendications 1 à 8 pour utilisation en médecine.

10. Composé selon l'une quelconque des revendications 1 à 8 pour utilisation dans le traitement du psoriasis.

11. Composition pharmaceutique comprenant un composé selon l'une quelconque des revendications 1 à 8 et un support pharmaceutiquement acceptable.

12. Procédé de préparaton d'un composé tel que défini selon l'une quelconque des revendications 1 à 8, ledit procédé comprenant la réaction d'un composé de formule II 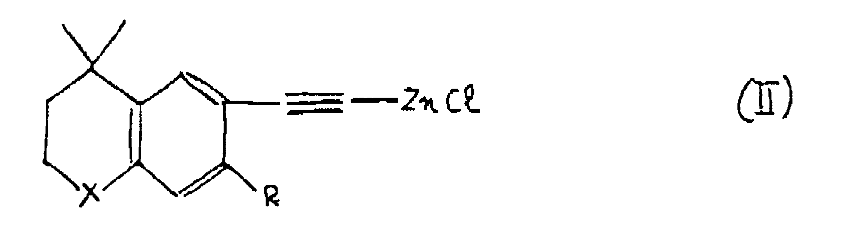 ,en présence de Pd(PQ₃)₄ (Q étant un phényle) ou d'un complexe similaire, avec un composé de formule III 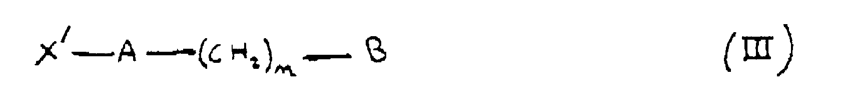 formules II et III dans lesquelles X,R,A et n sont tels que définis dans les revendications 1 a 8, B est un acide, un alcool, un aldéhyde ou une cétone prolégés, et X' est un halogène, ladite réaction étant suivie, s'il est nécessaire, par la libération, de sa protection, d'un groupe B protégé, et si on la désire, par la conversion d'un composé de formule I en un autre composé de formule I.

13. Utilisation d'un composé de formule I tel que défini dans l'une quelconque des revendications 1 à 8, dans la fabrication d'un médicament pour le traitement du psoriasis.

## Revendications (Revendications pour l'(les) Etat(s) contractant(s) suivant(s): ES, GR)

1. Procédé de préparation d'un composé de formule I: 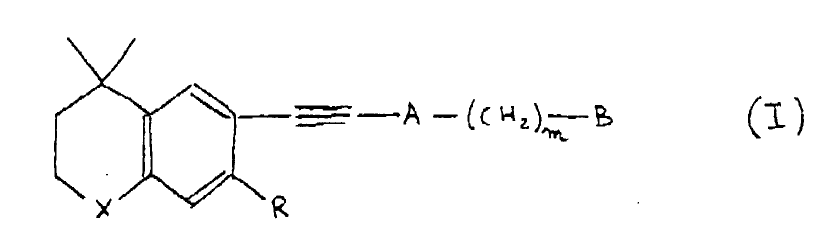 ou de l'un de ses sels, alkyléthers, esters ou amides formule dans laquelle :
X est S, O ou NR', R' étant l'hydrogène ou un alkyle en C₁ à C₆;
R est l'hydrogène ou un alkyle en C₁ à C₆;
A est un pyridyle, un thiényle, un furyle, un pyridazinyle, un pyrimidinyle ou un pyrazinyle;
n est de 0 à 5;
B est l'hydrogène, CO₂H, CH₂OH, CHO ou un de leurs dérivés alkyl-acétals en C₁ à C₆, ou COR₁ ou un de ses dérivés alkyl-cétals en C₁ à C₆, dans lequel R1 est (CH₂)ₘCH₃ et m est égal à 0 à 4 ;
et dans lequel l'ester est dérivé d'un alcool ou acide aliphatique saturé présentant jusqu'à 10 atomes de carbone, un alcool ou acide cycloaliphatique cyclique ou saturé présentant de 5 à 10 atomes de carbone, ou est un ester phénylique ou phénylique alkylé à 1 à 6 atomes de carbone et dans lequel l'amide est un amide non substitué ou un amide mono ou di-substitué dans lequel le substituant est un radical alipathique saturé présentant jusqu'à 10 atomes de carbone, ou un radical cycloaliphatique saturé ou cyclique présentant de 5 à 10 atomes de carbone ou un radical phénylique ou phénylique alkylé à un à six atomes de carbone, ledit procédé comprenant la réaction d'un composé de formule II : 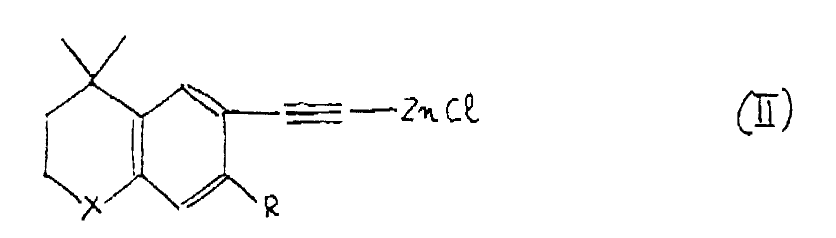 , en présence de Pd(PQ₃)₄ (Q étant un phényle) ou d'un complexe similaire, avec un composé de formule III 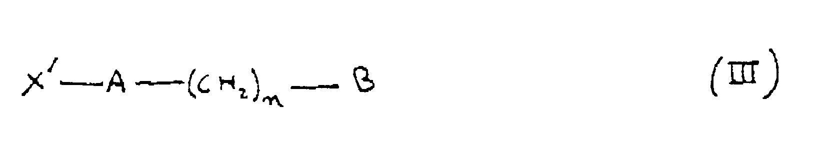 formules II et III dans lesquelles X,R,A et n sont tels que définis dans les revendications 1 à 8, B est un acide, un alcool, un aldéhyde ou une cétone protégés, et X' est un halogène, ladite réaction étant suivie, s'il est nécessaire, par la libération, de sa protection, d'un groupe B protégé, et si on la désire, par la conversion d'un composé de formule I en un autre composé de formule I.

2. Procédé de préparation d'un composé suivant la revendication 1 dans lequel n est égal à zéro.

3. Procédé de préparation d'un composé suivant la revendication 1 dans lequel n est égal à 1,2 ou 3.

4. Procédé selon l'une quelconque des revendications 1 à 3 pour la préparation d'un composé dans lequel X est S ou O, A est un pyridyle et B est -COOH, un de ses sels, esters ou amides pharmaceutiquement acceptables.

5. Procédé selon la revendication 4 pour la préparation d'un composé qui est le [(diméthyl-4,4-thiochromanyl-6)-2-acétylényl]-6-nicotinate d'éthyle, l'acide [(diméthyl-4,4-thiochromanyl-6)-2-acéty-lényl]-6-nicotonique, le (diméthyl-4,4-chromanyl-6)-2-acéty-lényl]-6-nicotinate d'éthyle et l'acide [(diméthyl-4,4- chromanyl-6)-2-acétylényl]-6-nicotinique ou un sel pharmaceutiquement acceptable de ceux-ci.

6. Procédé selon l'une quelconque des revendications 1 à 3 pour la préparation d'un composé dans lequel X est S ou O, A est un thiényle et B est -COOH ou un de ses sels, esters ou amides, pharmaceutiquement acceptables.

7. Procédé selon l'une quelconque des revendications 1 à 3 pour la préparation d'un composé dans lequel X est S ou O, A est un furyle et B est -COOH ou un de ses sels, esters ou amides, pharmaceutiquement acceptables.

8. Procédé selon l'une quelconque des revendications 1 à 3 pour la préparation d'un composé dans lequel X est S ou O, A est un pyridazinyle, pyrimidinyle ou pyrazinyle et B est -COOH ou un de ses sels, esters ou amides, pharmaceutiquement acceptables.

9. Procédé selon l'une des revendications 1 à 8 caractérisé en ce qu'on fait réagir le composé ayant la formule II avec le composé ayant la formule III en présence de Pd(PQ₃)₄, Q étant un phényle.

10. Procédé de préparation d'une composition pharmaceutique consistant à associer un composé défini dans l'une quelconque des revendications 1 à 8 à un support pharmaceutiquement acceptable.

11. Utilisation d'un composé tel que défini dans l'une quelconque des revendications 1 à 8, dans la fabrication d'un médicament pour le traitement du psoriasis.

## Patentansprüche (Patentansprüche für folgende(n) Vertragsstaat(en): AT, BE, CH, DE, FR, GB, IT, LI, LU, NL, SE)

1. Verbindung der Formel I: 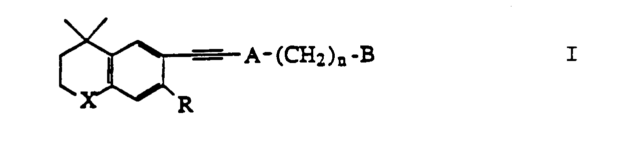 oder ein Salz, ein Alkyläther, Ester oder Amid hiervon, wobei X bedeutet S, O oder NR', worin R' Wasserstoff oder C₁₋₆ Alkyl ist; R ist Wasserstoff oder C₁₋₆ Alkyl; A ist Pyridyl, Thienyl, Furyl, Pyridazinyl, Pyrimidinyl oder Pyrazinyl; n ist 0-5; B ist H, CO₂H, CH₂OH, CHO oder ein C₁₋₆ Alkylazetalderivat hiervon, COR₁ oder ein C₁₋₆ Alkylketalderivat hiervon, worin R₁ (CH₂)ₘCH₃ und m 0-4 ist, und worin der Ester von einem gesättigten aliphatischen Alkohol oder einer Säure mit bis zu zehn Kohlenstoffatomen, einem zyklischen oder gesättigten aliphatisch zyklischen Alkohol oder Säure von 5 bis 10 Kohlenstoffatomen abgeleitet ist, oder ein Phenyl oder C₁₋₆ Alkylphenyl Ester ist; und das Amid ein nicht-substituiertes Amid oder ein mono- oder di-substituiertes Amid ist, in welchem der Substituent ein gesättigtes aliphatisches Radikal mit bis zu 10 Kohlenstoffatomen, oder ein zyklisches oder gesättigtes aliphatisch-zyklisches Radikal mit 5 bis 10 Kohlenstoffatomen oder ein Phenyl oder C₁₋₆ Alkylphenyl-Radikal ist.

2. Die Verbindung nach Anspruch 1, wobei n den Wert 0 besitzt.

3. Die Verbindung nach Anspruch 1, wobei n den Wert 1, 2 oder 3 besitzt.

4. Verbindung nach einem der Ansprüche 1 bis 3, wobei X ist S oder O, A ist Pyridyl und B ist -COOH, ein pharmazeutisch verträgliches Salz hiervon oder ein Ester oder Amid hiervon.

5. Verbindung nach Anspruch 4, bei der es sich um Äthyl-6-[2-(4,4-dimethylthiochroman-6-yl)ethynyl]nicotinoat, 6-[2-(4,4-Dimethylthiochroman-6-yl)ethynyl]nicotinsäure, Äthyl 6-[2-(4,4-dimethylchroman-6-yl)ethynyl]nicotinoat oder 6-[2-(4,4-Dimethylchroman-6-yl)ethynyl]nicotinsäure oder um ein pharmazeutisch verträgliches Salz hiervon handelt.

6. Verbindung nach einem der Ansprüche 1 bis 3, wobei X ist S oder O, A ist Thienyl und B ist -COOH oder ein pharmazeutisch verträgliches Salz, Ester oder Amid hiervon.

7. Verbindung nach einem der Ansprüche 1 bis 3, wobei X ist S oder O, A ist Furyl und B ist -COOH oder ein pharmazeutisch verträgliches Salz, Ester oder Amid hiervon.

8. Verbindung nach einem der Ansprüche 1 bis 3, wobei X ist S oder O, A ist Pyridazinyl, Pyrimidinyl oder Pyrazinyl und B ist -COOH oder ein pharmazeutisch verträgliches Salz, Ester oder Amid hiervon.

9. Verbindung nach einem der Ansprüche 1 bis 8 zur Verwendung in der Medizin.

10. Verbindung nach einem der Ansprüche 1 bis 8 zur Verwendung bei der Behandlung von Psoriase.

11. Pharmazeutische Zusammensetzung, welche eine in einem der Ansprüche 1 bis 8 definierte Verbindung und einen pharmazeutisch verträglichen Träger umfaßt.

12. Verfahren zur Herstellung einer Verbindung, wie in einem der Ansprüche 1 bis 8 definiert, das Verfahren umfassend Reaktion einer Verbindung der Formel II: 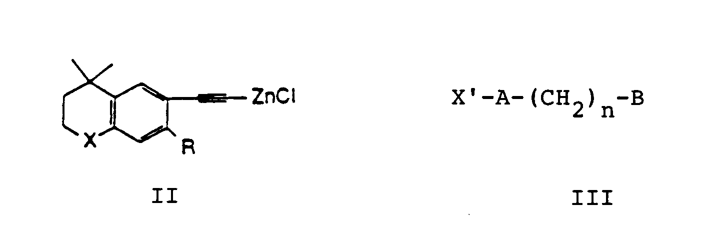 mit einer Verbindung der Formel III, in Gegenwart von Pd (PQ₃)₄ (Q ist Phenyl) oder einem ähnlichen Komplex, worin X, R, A, n wie in den Ansprüchen 1 bis 8 definiert sind, B eine geschützte Säure, Alkohol, Aldehyd oder Keton ist und X' ein Halogen ist, und danach falls erforderlich Entschützen einer geschützten Gruppe B, sowie, falls erwünscht, Umwandeln einer Verbindung der Formel I in eine andere Verbindung der Formel I.

13. Verwendung einer Verbindung der Formel I wie in einem der Ansprüche 1 bis 8 definiert zur Herstellung eines Medikaments für die Behandlung von Psoriase.

## Patentansprüche (Patentansprüche für folgende(n) Vertragsstaat(en): ES, GR)

1. Verfahren zur Herstellung einer Verbindung der Formel I: 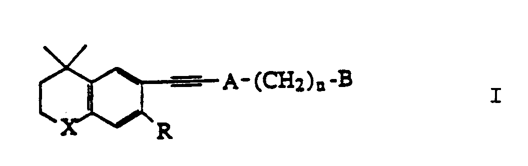 oder eines Salzes, Alkyläthers, Esters oder Amids hiervon, worin X bedeutet S, O oder NR', worin R' Wasserstoff oder C₁₋₆ Alkyl ist; R ist Wasserstoff oder C₁₋₆Alkyl; A ist Pyridyl, Thienyl, Furyl, Pyridazinyl, Pyrimidinyl oder Pyrazinyl; n ist 0-5; B ist H, Co₂H, CH₂OH, CHO oder ein C₁₋₆ Alkylazetalderivat hiervon, COR₁ oder ein C₁₋₆ Alkylketalderivat hiervon, worin R₁ (CH₂)ₘCH₃ und m 0-4 ist, und worin der Ester von einem gesättigten aliphatischen Alkohol oder einer Säure mit bis zu zehn Kohlenstoffatomen, einem zyklischen oder gesättigten aliphatisch zyklischen Alkohol oder Säure von 5 bis 10 Kohlenstoffatomen abgeleitet ist, oder ein Phenyl oder C₁₋₆ Alkylphenyl Ester ist; und das Amid ein nicht-substituiertes Amid oder ein mono- oder di-substituiertes Amid ist, in welchem der Substituent ein gesättigtes aliphatisches Radikal mit bis zu 10 Kohlenstoffatomen, oder ein zyklisches oder gesättigtes aliphatisch-zyklisches Radikal mit 5 bis 10 Kohlenstoffatomen oder ein Phenyl oder C₁₋₆ Alkylphenyl-Radikal, das genannte Verfahren umfassend Reaktion einer Verbindung der Formel II: 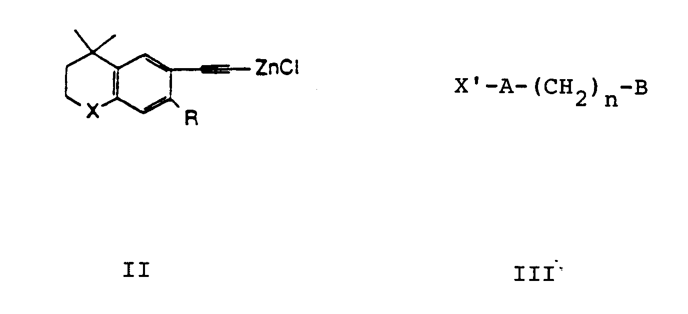 mit einer Verbindung der Formel III, in Gegenwart von Pd (PQ₃)₄ (Q ist Phenyl) oder einem ähnlichen Komplex worin X, R, A, n wie oben definiert sind, B ist H oder eine geschützte Säure, Alkohol, Aldehyd oder Keton ist und X' ein Halogen ist, und falls erforderlich Entschützen einer geschützten Gruppe B, sowie, falls erwünscht, Umwandeln einer Verbindung der Formel I in eine andere Verbindung der Formel I.

2. Verfahren nach Anspruch 1 zur Herstellung einer Verbindung, wobei n den Wert 0 besitzt.

3. Verfahren nach Anspruch 1 zur Herstellung einer Verbindung, wobei n den Wert 1, 2 oder 3 besitzt.

4. Verfahren nach einem der Ansprüche 1 bis 3 zur Herstellung einer Verbindung, bei welcher X ist S oder O, A ist Pyridyl und B ist -COOH, ein pharmazeutisch verträgliches Salz hiervon oder ein Ester oder Amid hiervon.

5. Verfahren nach Anspruch 4 zur Herstellung einer Verbindung, bei der es sich um Äthyl-6-[2-(4,4-dimethylthiochroman-6-yl)ethynyl]nicotinoat, 6-[2-(4,4-Dimethylthiochroman-6-yl)ethynyl]nicotinsäure, Äthyl 6-[2-(4,4-dimethylchroman-6-yl)ethynyl]nicotinoat oder 6-[2-(4,4-Dimethylchroman-6yl)ethynyl]nicotinsäure oder um ein pharmazeutisch verträgliches Salz hiervon handelt.

6. Verfahren nach einem der Ansprüche 1 bis 3 zur Herstellung einer Verbindung, wobei X ist S oder O, A ist Thienyl und B ist -COOH oder ein pharmazeutisch verträgliches Salz, Ester oder Amid hiervon.

7. Verfahren nach einem der Ansprüche 1 bis 3 zur Herstellung einer Verbindung, bei welcher X ist S oder O, A ist Furyl und B ist -COOH oder ein pharmazeutisch verträgliches Salz, Ester oder Amid hiervon.

8. Verfahren nach einem der Ansprüche 1 bis 3 zur Herstellung einer Verbindung, bei welcher X ist S oder O, A ist Pyridazinyl, Pyrimidinyl oder Pyrazinyl und B ist -COOH oder ein pharmazeutisch verträgliches Salz, Ester oder Amid hiervon.

9. Verfahren nach einem der Ansprüche 1 bis 8, wobei die Verbindung der Formel II mit der Verbindung der Formel III in Gegenwart von Pd(PQ₃)₄ zur Reaktion gebracht wird, wobei Q Phenyl ist.

10. Verfahren zur Herstellung einer pharmazeutischen Zusammensetzung, bei welchem eine Verbindung der in einem der Ansprüche 1 bis 8 definierten Art in Verbund mit einem pharmazeutisch verträglichen Träger gebracht wird.

11. Verwendung einer Verbindung wie in einem der Ansprüche 1 bis 8 definiert, zur Herstellung eines Medikaments für die Behandlung von Psoriase.
